# EUROPEAN PATENT SPECIFICATION

(11) **EP 4 337 302 B1**
(45) Date of publication and mention of the grant of the patent: **08.04.2026**
(21) Application number: 22722655.2
(22) Date of filing: 26.04.2022
(51) Int. Cl.: A61N 1/36

(54) **REMOTE TITRATION OF NEUROSTIMULATION THERAPY**
FERNTITRATION EINER NEUROSTIMULATIONSTHERAPIE
TITRAGE À DISTANCE D'UNE THÉRAPIE PAR NEUROSTIMULATION

(30) Priority: 10.05.2021 US 202163186596 P
(43) Date of publication of application: 20.03.2024
(60) Divisional application: 26154903.4
(73) Proprietor: Boston Scientific Neuromodulation Corporation, Valencia, CA 91355 (US)
(72) Inventor: MOORE, Lisa Denise, Glendale, California 91214 (US); MUSTAKOS, Richard, Simi Valley, California 93063 (US)
(74) Representative: Vossius & Partner Patentanwälte Rechtsanwälte mbB
(86) International application number: PCT/US2022/026311
(87) International publication number: WO 2022/240580

(56) References cited:
- US-A1- 2014 350 636
- US-A1- 2021 008 371

## Description

### TECHNICAL FIELD

This document relates generally to medical devices, and more particularly, to systems, devices and methods for determining and setting of stimulation parameters for programming an electrical stimulation system.

### BACKGROUND

Neuromodulation (or "neural neuromodulation", also referred to as "neurostimulation" or "neural stimulation") has been proposed as a therapy for a number of conditions. Often, neuromodulation and neural stimulation may be used interchangeably to describe excitatory stimulation that causes action potentials as well as inhibitory and other effects. Examples of neuromodulation include Spinal Cord Stimulation (SCS), Deep Brain Stimulation (DBS), Peripheral Nerve Stimulation (PNS), and Functional Electrical Stimulation (FES). SCS systems have been used as a therapeutic modality for the treatment of chronic pain syndromes. PNS has been used to treat chronic pain syndrome and incontinence, with a number of other applications under investigation. FES systems have been applied to restore some functionality to paralyzed extremities in spinal cord injury patients. DBS can be used to treat a variety of diseases or disorders.

Stimulation systems, such as implantable electrostimulators, have been developed to provide therapy for a variety of treatments. An implantable electrostimulator can include a pulse generator and one or more leads each including a plurality of stimulation electrodes. The stimulation electrodes are in contact with or near target tissue to be stimulated, such as nerves, muscles, or other tissue. The control module generates a control signal to the pulse generator, which generates electrostimulation pulses that are delivered by the electrodes to the target tissue in accordance with an electrode configuration and a set of stimulation parameters.

Prior art is known from US 2021/008371 A1 and US 2014/350636 A1.

### SUMMARY

Various examples discussed in this document may provide more effective control of neuromodulation therapy such as deep brain stimulation (DBS). In accordance with various examples discussed in this document, a pre-generated stimulation program may be modified by a user such as the patient with the use of a remotely controlled device in an in-home therapy titration session. The remotely controlled in-home therapy titration allows the user to customize the stimulation settings to better address individual patient's need such as at different patient states or under evolving patient medical conditions.

The description that follows will generally focus on the use of the invention within a DBS system, such as that disclosed in U.S. Patent Application Publication 2020/0001091. However, the present invention may find applicability with any implantable neurostimulator device system, including Spinal Cord Stimulation (SCS) systems, Vagus Nerve Stimulation (VNS) system, Sacral Nerve Stimulation (SNS) systems, and the like.

Further details about the present subject matter are found in the detailed description and appended claims. Other aspects of the disclosure will be apparent to persons skilled in the art upon reading and understanding the following detailed description and viewing the drawings that form a part thereof, each of which are not to be taken in a limiting sense. The scope of the present invention is defined by the appended claims.

### BRIEF DESCRIPTION OF THE DRAWINGS

Various examples are illustrated by way of example in the figures of the accompanying drawings. Such examples are demonstrative and not intended to be exhaustive or exclusive examples of the present subject matter.
FIG. 1 illustrates, by way of example and not limitation, an electrical stimulation system that may be used to deliver deep brain stimulation (DBS).
FIG. 2 illustrates, by way of example and not limitation, an implantable pulse generator (IPG) that may be used in a DBS system.
FIGS. 3A-3B illustrate, by way of example and not limitation, leads that may be coupled to an IPG to deliver electrostimulation such as DBS.
FIG. 4 illustrates, by way of example and not limitation, a computing device for programming or controlling the operation of an electrostimulation system.
FIG. 5 illustrates, by way of example and not limitation, a stimulation parameter control system and a part of the environment in which it may operate.
FIG. 6A illustrates, by way of example and not limitation, a graphical user interface (GUI) for programming a stimulation setting into an IPG.
FIG. 6B illustrates, by way of example and not limitation, a diagram of an electrode configuration and parameter search space.
FIG. 7 illustrates, by way of example and not limitation, an example of an in-home therapy titration system for modifying a pre-generated base stimulation program for DBS.
FIG. 8 is a flow chart illustrating, by way of example and not limitation, a method for creating a base stimulation program and titrating such program in an in-home titration session and providing electrostimulation to a neural target.
FIG. 9 illustrates generally a block diagram of an example machine 900 upon which any one or more of the techniques (e.g., methodologies) discussed herein may perform.

### DETAILED DESCRIPTION

This document describes systems and methods for creating, maintaining, and remotely modifying stimulation settings for a neuromodulation therapy such as deep brain stimulation. According to an example, a neurostimulation system includes an implantable stimulator to provide electrostimulation via a lead comprising a plurality of electrodes, and a programming device. The programing device identifies, for the lead, a search space of electrode configurations and parameter values, and determines, based on a clinical response indicator under a first patient state, at least one base stimulation setting including an optimal electrode configuration and an optimal stimulation parameter value from the identified search space. The system includes a remotely controlled therapy titration device that can modify the base stimulation setting under a second patient state based on an evaluation of a modified clinical response indicator. The modified base stimulation setting may be stored in the memory, or used by the implantable stimulator to deliver electrostimulation.

Various examples described herein involve deep brain stimulation (DBS). The following detailed description of the present subject matter refers to the accompanying drawings which show, by way of illustration, specific aspects and examples in which the present subject matter may be practiced. These examples are described in sufficient detail to enable those skilled in the art to practice the present subject matter. Other examples may be utilized and structural, logical, and electrical changes may be made without departing from the scope of the present subject matter. References to "an", "one", or "various" examples in this disclosure are not necessarily to the same example, and such references contemplate more than one example. The following detailed description is, therefore, not to be taken in a limiting sense, and the scope is defined only by the appended claims.

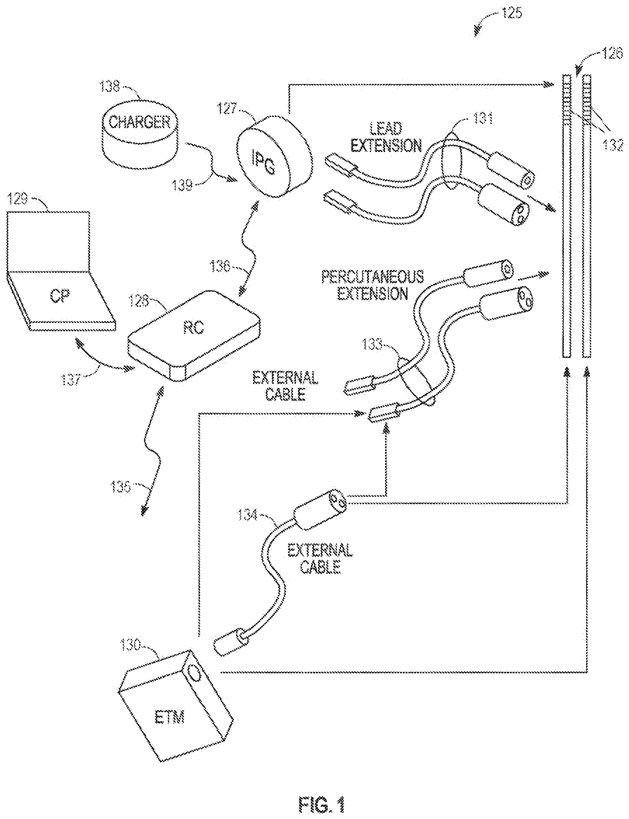

FIG. 1 illustrates, by way of example, an example of an electrical stimulation system 100, which may be used to deliver DBS. The electrical stimulation system 100 may generally include a one or more (illustrated as two) of implantable neuromodulation leads 126, an implantable pulse generator (IPG) 127, an external remote controller (RC) 128, a clinician programmer (CP) 129, and an external trial modulator (ETM) 130. The IPG 127 may be physically connected via one or more percutaneous lead extensions 131 to the neuromodulation lead(s) 126, which carry a plurality of electrodes 132. The electrodes, when implanted in a patient, form an electrode arrangement. As illustrated, the neuromodulation leads 126 may be percutaneous leads with the electrodes arranged in-line along the neuromodulation leads or about a circumference of the neuromodulation leads. Any suitable number of neuromodulation leads can be provided, including only one, as long as the number of electrodes is greater than two (including the IPG case function as a case electrode) to allow for lateral steering of the current. Alternatively, a surgical paddle lead can be used in place of one or more of the percutaneous leads. The IPG 127 includes pulse generation circuitry that delivers electrical modulation energy in the form of a pulsed electrical waveform (*i.e.,* a temporal series of electrical pulses) to the electrodes in accordance with a set of modulation parameters.

The ETM 130 may also be physically connected via the percutaneous lead extensions 133 and external cable 134 to the neuromodulation lead(s) 126. The ETM 130 may have similar pulse generation circuitry as the IPG 127 to deliver electrical modulation energy to the electrodes in accordance with a set of modulation parameters. The ETM 130 is a non-implantable device that may be used on a trial basis after the neuromodulation leads 126 have been implanted and prior to implantation of the IPG 127, to test the responsiveness of the modulation that is to be provided. Functions described herein with respect to the IPG 127 can likewise be performed with respect to the ETM 130.

The RC 128 may be used to telemetrically control the ETM 130 via a bi-directional RF communications link 135. The RC 128 may be used to telemetrically control the IPG 127 via a bi-directional RF communications link 136. Such control allows the IPG 127 to be turned on or off and to be programmed with different modulation parameter sets. The IPG 127 may also be operated to modify the programmed modulation parameters to actively control the characteristics of the electrical modulation energy output by the IPG 127. A clinician may use the CP 129 to program modulation parameters into the IPG 127 and ETM 130 in the operating room and in follow-up sessions.

The CP 129 may indirectly communicate with the IPG 127 or ETM 130, through the RC 128, via an IR communications link 137 or another link. The CP 129 may directly communicate with the IPG 127 or ETM 130 via an RF communications link or other link (not shown). The clinician detailed modulation parameters provided by the CP 129 may also be used to program the RC 128, so that the modulation parameters can be subsequently modified by operation of the RC 128 in a stand-alone mode (i.e., without the assistance of the CP 129). Various devices may function as the CP 129. Such devices may include portable devices such as a lap-top personal computer, mini-computer, personal digital assistant (PDA), tablets, phones, or a remote control (RC) with expanded functionality. Thus, the programming methodologies can be performed by executing software instructions contained within the CP 129. Alternatively, such programming methodologies can be performed using firmware or hardware. In any event, the CP 129 may actively control the characteristics of the electrical modulation generated by the IPG 127 to allow the desired parameters to be determined based on patient feedback or other feedback and for subsequently programming the IPG 127 with the desired modulation parameters. To allow the user to perform these functions, the CP 129 may include user input device (e.g., a mouse and a keyboard), and a programming display screen housed in a case. In addition to, or in lieu of, the mouse, other directional programming devices may be used, such as a trackball, touchpad, joystick, touch screens or directional keys included as part of the keys associated with the keyboard. An external device (e.g. CP) may be programmed to provide display screen(s) that allow the clinician to, among other functions, select or enter patient profile information (e.g., name, birth date, patient identification, physician, diagnosis, and address), enter procedure information (e.g., programming/follow-up, implant trial system, implant IPG, implant IPG and lead(s), replace IPG, replace IPG and leads, replace or revise leads, explant, etc.), generate a pain map of the patient, define the configuration and orientation of the leads, initiate and control the electrical modulation energy output by the neuromodulation leads, and select and program the IPG with modulation parameters, including electrode selection, in both a surgical setting and a clinical setting. The display screen(s) may be used to suggest the electrode(s) for use to stimulate a targeted dorsal root. The external device(s) (e.g. CP and/or RC) may be configured to communicate with other device(s), including local device(s) and/or remote device(s). For example, wired and/or wireless communication may be used to communicate between or among the devices.

An external charger 138 may be a portable device used to transcutaneous charge the IPG 127 via a wireless link such as an inductive link 136. Once the IPG 127 has been programmed, and its power source has been charged by the external charger or otherwise replenished, the IPG 127 may function as programmed without the RC 128 or CP 129 being present.

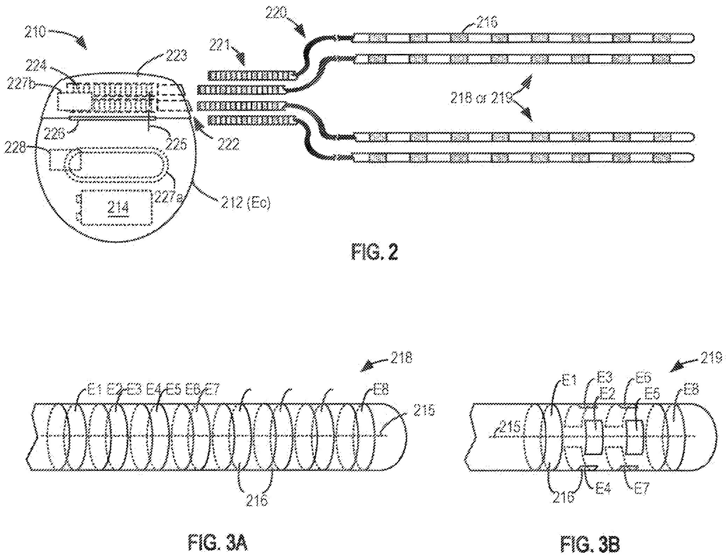

FIG. 2 illustrates, by way of example and not limitation, an IPG 210 in a DBS system. The IPG 210, which is an example of the IPG 117 of the electrical stimulation system 100 as illustrated in FIG. 1, may include a biocompatible device case 212 that holds the circuitry and a battery 214 for providing power for the IPG 210 to function, although the IPG 210 can also lack a battery and can be wirelessly powered by an external source. The IPG 210 may be coupled to one or more leads, such as leads 218 or 219 as illustrated herein. The lead 218 or 219 can each include a plurality of electrodes 216 for delivering electrostimulation energy, recording electrical signals, or both. In some examples, the leads 218 or 219 can be rotatable so that the electrodes 216 can be aligned with the target neurons after the neurons have been located such as based on the recorded signals.

The leads 218 or 219 can be implanted near or within the desired portion of the body to be stimulated. In an example of operations for DBS, access to the desired position in the brain can be accomplished by drilling a hole in the patient's skull or cranium with a cranial drill (commonly referred to as a burr), and coagulating and incising the dura mater, or brain covering. A lead can then be inserted into the cranium and brain tissue with the assistance of a stylet (not shown). The lead can be guided to the target location within the brain using, for example, a stereotactic frame and a microdrive motor system. In some examples, the microdrive motor system can be fully or partially automatic. The microdrive motor system may be configured to perform actions such as inserting, advancing, rotating, or retracing the lead.

Lead body of the leads 218 or 219 can be formed of a biocompatible, non-conducting material such as, for example, a polymeric material. Suitable polymeric materials include, but are not limited to, silicone, polyurethane, polyurea, polyurethane-urea, polyethylene, or the like. Once implanted in the body, the leads 218 or 219 may be in contact with body tissue for extended periods of time. In some examples, the leads 218 or 219 can have a cross-sectional diameter of no more than 1.5 mm and may be in the range of 0.5 to 1.5 mm.

The electrodes 216 can be made of metal, alloy, conductive oxide, or any other suitable conductive biocompatible material. Examples of suitable materials include, but are not limited to, platinum, platinum iridium alloy, iridium, titanium, tungsten, palladium, palladium rhodium, or the like. Preferably, the electrodes are made of a material that is biocompatible and does not substantially corrode under expected operating conditions in the operating environment for the expected duration of use. The electrodes 216 can include one or more ring electrodes, and/or one or more sets of segmented electrodes (or any other combination of electrodes), examples of which are discussed below with reference to FIGS. 3A and 3B.

Lead wires 220 within the leads may be coupled to the electrodes 216 and to proximal contacts 221 insertable into lead connectors 222 fixed in a header 223 on the IPG 210, which header can comprise an epoxy for example. Alternatively, the proximal contacts 221 may connect to lead extensions (not shown) which are in turn inserted into the lead connectors 222. Once inserted, the proximal contacts 221 connect to header contacts 224 within the lead connectors 222, which are in turn coupled by feedthrough pins 225 through a case feedthrough 226 to stimulation circuitry 228 within the case 212, which stimulation circuitry 228 is described below.

By way of example and not limitation, the IPG 210 illustrated in FIG. 2 can be coupled to four percutaneous leads 218 or 219 (218 is shown), and thus the header 223 may include a 2x2 array of eight-electrode lead connectors 222. However, the type and number of leads, and the number of electrodes, in an IPG is application specific and therefore can vary. In another example not shown, a given lead can have sixteen electrodes, and thus this lead would have two sets of proximal contacts 221 to mate with two of the eight-electrode lead connectors 222, as disclosed for example in U.S. Patent Application Publication 2019/0076645. The conductive case 212 can also comprise an electrode (Ec).

In a DBS application, as is useful in the treatment of tremor in Parkinson's disease for example, the IPG 210 is typically implanted under the patient's clavicle (collarbone). The leads 218 or 219 (which may be extended by lead extensions, not shown) can be tunneled through and under the neck and the scalp, with the electrodes 216 implanted through holes drilled in the skull and positioned for example in the subthalamic nucleus (STN) and the pedunculopontine nucleus (PPN) in each brain hemisphere. The IPG 210 can also be implanted underneath the scalp closer to the location of the electrodes' implantation. The leads 218 or 219, or the extensions 633, can be integrated with and permanently connected to the IPG 210 in other solutions.

The IPG 210 can include an antenna 227a allowing it to communicate bi-directionally with a number of external devices discussed subsequently. The antenna 227a as shown comprises a conductive coil within the case 212, although the coil of the antenna 227a can also appear in the header 223. When the antenna 227a is configured as a coil, communication with external devices preferably occurs using near-field magnetic induction. The IPG 210 may also include a Radio-Frequency (RF) antenna 227b. Although the RF antenna 227b is shown within the header 223, in some examples it may also be within the case 212. The RF antenna 227b may comprise a patch, slot, or wire, and may operate as a monopole or dipole. The RF antenna 227b preferably communicates using far-field electromagnetic waves, and may operate in accordance with any number of known RF communication standards, such as Bluetooth, Zigbee, WiFi, MICS, and the like. If the IPG 210 lacks a battery 214, an additional coil can be present to receive wireless power from an external source.

Stimulation in IPG 210 is typically provided by pulses each of which may include one phase or multiple phases. For example, a monopolar stimulation current can be delivered between a lead-based electrode (e.g., one of the electrodes 216) and the case electrode Ec 212. A bipolar stimulation current can be delivered between two lead-based electrodes (e.g., two of the electrodes 216). Stimulation parameters typically include current amplitude (or voltage amplitude), frequency, pulse width of the pulses or of its individual phases; electrodes selected to provide the stimulation; polarity of such selected electrodes, i.e., whether they act as anodes that source current to the tissue, or cathodes that sink current from the tissue. Each of the electrodes can either be used (an active electrode) or unused (OFF). When the electrode is used, the electrode can be used as an anode or cathode and carry anodic or cathodic current. In some instances, an electrode might be an anode for a period of time and a cathode for a period of time. These and possibly other stimulation parameters taken together comprise a stimulation program that the stimulation circuitry 228 in the IPG 210 can execute to provide therapeutic stimulation to a patient.

In some examples, a measurement device coupled to the muscles or other tissue stimulated by the target neurons, or a unit responsive to the patient or clinician, can be coupled to the IPG 210 or microdrive motor system. The measurement device, user, or clinician can indicate a response by the target muscles or other tissue to the stimulation or recording electrode(s) to further identify the target neurons and facilitate positioning of the stimulation electrode(s). For example, if the target neurons are directed to a muscle experiencing tremors, a measurement device can be used to observe the muscle and indicate changes in, for example, tremor frequency or amplitude in response to stimulation of neurons. Alternatively, the patient or clinician can observe the muscle and provide feedback.

FIGS. 3A-3B illustrate, by way of example and not limitation, leads 216 and 218 that may be coupled to the IPG 210 to deliver electrostimulation such as DBS. FIG. 3A shows a lead 218 with electrodes 216 disposed at least partially about a circumference of the lead 218. The electrodes 216 may be located along a distal end portion of the lead 218. As illustrated herein, the electrodes 216 are ring electrodes that span 360 degrees about a circumference of the lead 218. A ring electrode allows current to project equally in every direction from the position of the electrode, and typically does not enable stimulus current to be directed from only a particular angular position or a limited angular range around of the lead. The lead 218, which includes only ring electrodes, is also referred to as a non-directional lead.

FIG. 3B shows a lead 219 with electrodes 216 including ring electrodes such as E1 at a proximal end and E8 at the distal end. Additionally, the lead 219 also include a plurality of segmented electrodes (also known as split-ring electrodes). For example, a set of segmented electrodes E2, E3, and E4 are around the circumference at a longitudinal position, each spanning less than 360 degrees around the lead axis. In an example, each of electrodes E2, E3, and E4 spans 90 degrees, with each being separated from the others by gaps of 30 degrees. Another set of segmented electrodes E5, E6, and E7 are located around the circumference at another longitudinal position different from the segmented electrodes E2, E3 and E4. Segmented electrodes such as E2-E7 can direct stimulus current to a selected angular range around the lead.

Segmented electrodes can typically provide superior current steering than ring electrodes because target structures in DBS or other stimulation are not typically symmetric about the axis of the distal electrode array. Instead, a target may be located on one side of a plane running through the axis of the lead. Through the use of a radially segmented electrode array, current steering can be performed not only along a length of the lead but also around a circumference of the lead. This provides precise three-dimensional targeting and delivery of the current stimulus to neural target tissue, while potentially avoiding stimulation of other tissue. In some examples, segmented electrodes can be together with ring electrodes. The lead 219, which include at least one or more segmented electrodes, is also referred to as a directional lead. In an example, all electrodes on a directional lead can be segmented electrodes. In another example, there can be different numbers of segmented electrodes at different longitudinal positions.

As illustrated in FIG. 3B, segmented electrodes may be grouped into sets of segmented electrodes, where each set is disposed around a circumference at a particular longitudinal location of the directional lead 219. The directional lead 219 may have any number (e.g., three as shown in FIG. 3B) segmented electrodes in a given set of segmented electrodes. By way of example and not limitation, a given set may include any number between two to 16 segmented electrodes. In an example, all sets of segmented electrodes may contain the same number of segmented electrodes. In another example, one set of the segmented electrodes may include a different number of electrodes than at least one other set of segmented electrodes.

The segmented electrodes may vary in size and shape. In some examples, the segmented electrodes are all of the same size, shape, diameter, width or area or any combination thereof. In some examples, the segmented electrodes of each circumferential set (or even all segmented electrodes disposed on the lead 219) may be identical in size and shape.

Each set of segmented electrodes may be disposed around the circumference of the lead body to form a substantially cylindrical shape around the lead body. The spacing between individual electrodes of a given set of the segmented electrodes may be the same, or different from, the spacing between individual electrodes of another set of segmented electrodes on the lead 219. In some examples, equal spaces, gaps or cutouts are disposed between each segmented electrode around the circumference of the lead 219. In other examples, the spaces, gaps or cutouts between the segmented electrodes may differ in size or shape. In other examples, the spaces, gaps, or cutouts between segmented electrodes may be uniform for a particular set of the segmented electrodes, or for all sets of the segmented electrodes. The sets of segmented electrodes may be positioned in irregular or regular intervals along a length the lead 219.

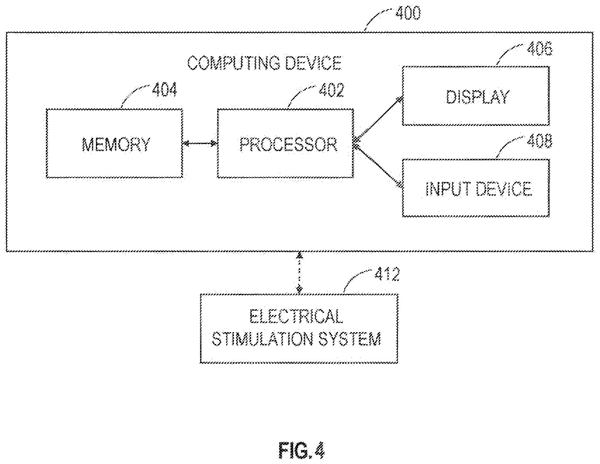

FIG. 4 illustrates, by way of example and not limitation, a computing device 400 for programming or controlling the operation of an electrical stimulation system 412. The computing device 400 includes a processor 402, a memory 404, a display 406, and an input device 408. Optionally, the computing device 400 may be separate from and communicatively coupled to the electrical stimulation system 412. Alternatively, the computing device 400 may be integrated with the electrical stimulation system 412. In an example, the computing device 400 can be a part of the electrical stimulation system 412, such as part of the IPG 127, RC 128, CP 129, or ETM 130 illustrated in FIG. 1.

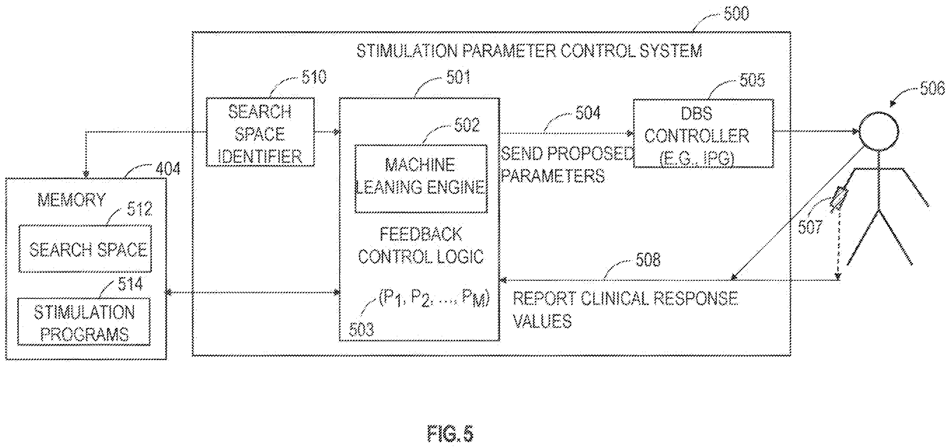

The computing device 400, also referred to as a programming device, can be a computer, tablet, mobile device, or any other suitable device for processing information. The computing device 400 can be local to the user or can include components that are non-local to the computer including one or both of the processor 402 or memory 404 (or portions thereof). For example, the user may operate a terminal that is connected to a non-local processor or memory. In some examples, the computing device 400 can include a watch, wristband, smartphone, or the like. Such computing devices can wirelessly communicate with the other components of the electrical stimulation system, such as the CP 129, RC 128, ETM 130, or IPG 127 illustrated in FIG. 1. The computing device 400 may be used for gathering patient information, such as general activity level or present queries or tests to the patient to identify or score pain, depression, stimulation effects or side effects, cognitive ability, or the like. In some examples, the computing device 400 may prompt the patient to take a periodic test (for example, every day) for cognitive ability to monitor, for example, Alzheimer's disease. In some examples, the computing device 400 may detect, or otherwise receive as input, patient clinical responses to electrostimulation such as DBS, and determine or update stimulation parameters using a closed-loop algorithm based on the patient clinical responses, as described below with reference to FIG. 5. Examples of the patient clinical responses may include physiological signals (e.g., heart rate) or motor parameters (e.g., tremor, rigidity, bradykinesia). The computing device 400 may communicate with the CP 129, RC 128, ETM 130, or IPG 127 and direct the changes to the stimulation parameters to one or more of those devices. In some examples, the computing device 400 can be a wearable device used by the patient only during programming sessions. Alternatively, the computing device 400 can be worn all the time and continually or periodically adjust the stimulation parameters. In an example, the closed-loop algorithm for determining or updating stimulation parameters can be implemented in a mobile device, such as a smartphone, that is connected to the IPG or an evaluating device (e.g. a wristband or watch). These devices can also record and send information to the clinician.

The processor 402 can include one or more processors that may be local to the user or non-local to the user or other components of the computing device 400. In an example, the processor 402 may execute instructions (e.g., stored in the memory 404) to determine a search space of electrode configurations and parameter values, create or update one or more stimulation settings that are selectable for use in electrostimulation therapies such as DBS. Such a search space refers to a collection of available electrodes, possible electrode configurations, and possible values or value ranges of one or more stimulation parameters that may be applied to selected electrodes to deliver electrostimulation. A stimulation setting includes an electrode configuration and values for one or more stimulation parameters. The electrode configuration may include information about electrodes (ring electrodes and/or segmented electrodes) selected to be active for delivering stimulation (ON) or inactive (OFF), polarity of the selected electrodes, electrode locations (e.g., longitudinal positions of ring electrodes along the length of a lead, or longitudinal positions and angular positions segmented electrodes on a circumference at a longitudinal position of the lead), stimulation modes such as monopolar pacing or bipolar pacing, etc. The stimulation parameters may include, for example, current amplitude values, current fractionalization across electrodes, stimulation frequency, stimulation pulse width, etc. The processor 402 can determine or modify a stimulation setting by searching through the search space through an optimization process, such as until an optimal, desired, or acceptable outcome is reached. Examples of generating a stimulation setting and providing electrostimulation therapy in accordance with the stimulation setting are discussed below with reference to FIG. 5.

In various examples, portions of the functions of the processor 402 may be implemented as a part of a microprocessor circuit. The microprocessor circuit can be a dedicated processor such as a digital signal processor, application specific integrated circuit (ASIC), microprocessor, or other type of processor for processing information. Alternatively, the microprocessor circuit can be a processor that can receive and execute a set of instructions of performing the functions, methods, or techniques described herein.

The memory 404 can store instructions executable by the processor 402 to perform varies functions including, for example, determining a reduced or restricted electrode configuration and parameter search space (also referred to as a "restricted search space"), creating or modifying one or more stimulation settings within the restricted search space, etc. The memory 404 may store the search space, and one or more stimulation programs each including respective stimulation settings each associated with respective optimization criteria such as clinical response indicators, as to be discussed with reference to FIG. 5.

The memory 404 can be a computer-readable storage media that includes, for example, nonvolatile, non-transitory, removable, and non-removable media implemented in any method or technology for storage of information, such as computer readable instructions, data structures, program modules, or other data. Examples of computer-readable storage media include RAM, ROM, EEPROM, flash memory, or other memory technology, CD-ROM, digital versatile disks ("DVD") or other optical storage, magnetic cassettes, magnetic tape, magnetic disk storage or other magnetic storage devices, or any other medium which can be used to store the desired information, and which can be accessed by a computing device.

Communication methods provide another type of computer readable media; namely communication media. Communication media typically embodies computer-readable instructions, data structures, program modules, or other data in a modulated data signal such as a carrier wave, data signal, or other transport mechanism and include any information delivery media. The terms "modulated data signal," and "carrier-wave signal" includes a signal that has one or more of its characteristics set or changed in such a manner as to encode information, instructions, data, and the like, in the signal. By way of example, communication media includes wired media such as twisted pair, coaxial cable, fiber optics, wave guides, and other wired media and wireless media such as acoustic, RF, infrared, Bluetooth.TM., near field communication, and other wireless media.

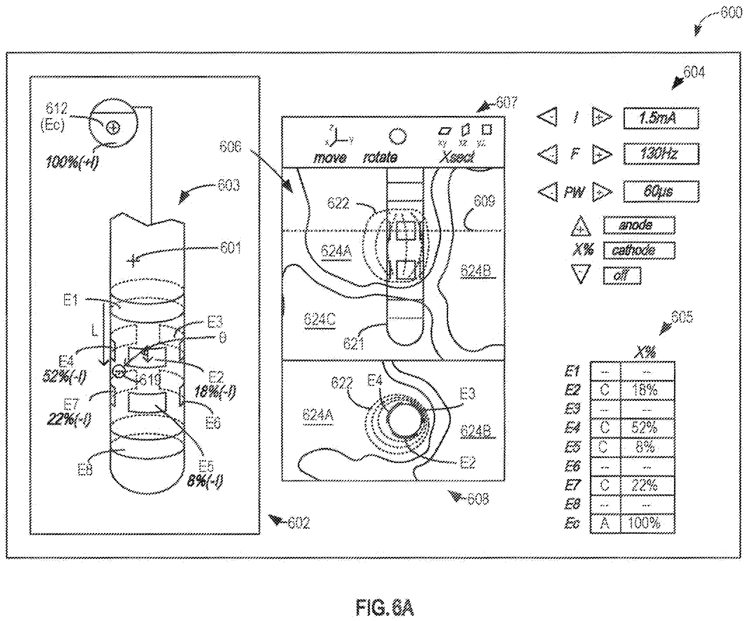

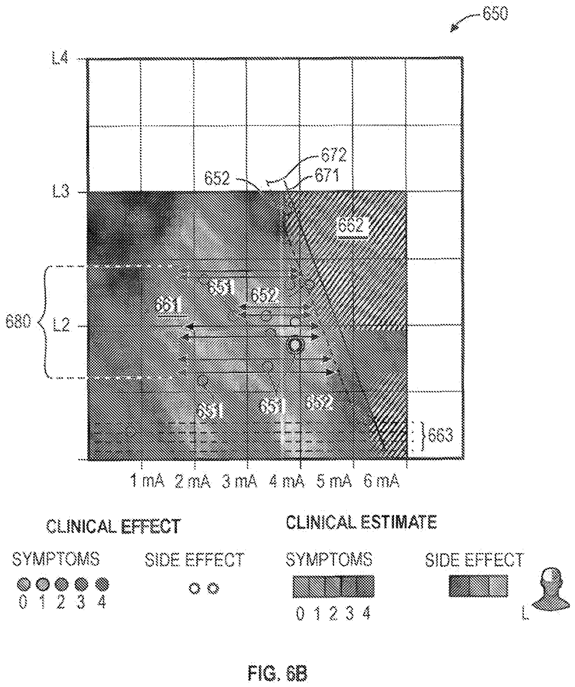

The display 406 can be any suitable display or presentation device, such as a monitor, screen, display, or the like, and can include a printer. The display 406 can be a part of a user interface configured to display information about stimulation settings (e.g., electrode configurations and stimulation parameter values and value ranges) and user control elements for programming a stimulation setting into an IPG such as that shown in FIG. 6A, the search space such as that shown in FIG. 6B, among others. In some examples, the user interface may include a speaker that provides audio information about stimulation settings, and receive audio input from the user, such as a clinician or the patient.

The input device 408 can be, for example, a keyboard, mouse, touch screen, track ball, joystick, voice recognition system, or any combination thereof, or the like. Another input device 408 can be a camera from which the clinician can observe the patient. Yet another input device 408 is a microphone where the patient or clinician can provide responses or queries.

The electrical stimulation system 412 can include, for example, any of the components illustrated in FIG. 1. The electrical stimulation system 412 may communicate with the computing device 400 through a wired or wireless connection or, alternatively or additionally, a user can provide information between the electrical stimulation system 412 and the computing device 400 using a computer-readable medium or by some other mechanism. As mentioned, in some examples, the computing device 400 may include part of the electrical stimulation system, such as, for example, the IPG 127, RC 128, CP 129, or ETM 130 or any combination thereof.

FIG. 5 illustrates, by way of example and not limitation, a stimulation parameter control system 500 and a part of the environment in which it may operate. The stimulation parameter control system 500, which may be implemented as a part of the processor 402, may include a feedback control logic 501, a DBS controller 505, and a search space identifier 510. The feedback control logic 501 may be implemented in, for example, the CP 129 or the RC 128. The feedback control logic 501 can determine or modify one or more stimulation settings 504 for a stimulation lead (such as leads 218 or 219) at target stimulation region, such as a region in a brain hemisphere. A stimulation setting includes electrode configuration and values for one or more stimulation parameters (P₁, P₂, ..., Pₘ) 503. The electrode configuration includes information about electrodes (ring electrodes and/or segmented electrodes) selected to be active for delivering stimulation (ON) or inactive (OFF), polarity of the selected electrodes, electrode locations (also referred to as contact locations, which may include longitudinal positions of ring electrodes along the length of a lead, or angular positions of segmented electrodes about a circumference at a longitudinal position of the lead), stimulation modes such as monopolar pacing or bipolar pacing, etc. The stimulation parameters may include, for example, current amplitude values, current fractionalization across electrodes, stimulation frequency, stimulation pulse width, etc. In some examples, the feedback control logic 501 may modify the stimulation setting 504 such as by changing a stimulation parameter value, or modifying an electrode configuration.

The stimulation setting 504 may be provided to the DBS controller 505 to configure the IPG 127 or ETM 130 to deliver DBS therapy to the patient 506 in accordance with the stimulation setting or the modified stimulation setting. The stimulation may produce certain therapeutic effects and/or side effects on the patient 506. Such therapeutic effectiveness and side effects, also referred to as clinical responses or clinical metrics, may be provided to the feedback control logic 501. In an example, the clinical responses may be based on patient or clinician observations. For example, motor symptoms such as bradykinesia (slowness of movement), rigidity, tremor, among other symptoms or side effects, can be scored by the patient or by the clinician upon overserving or questioning the patient. In some examples, the clinical responses can be objective in nature, such as measurements automatically or semi-automatically taken by a sensor 507. In an example, the sensor 507 may be included in a wearable device associated with patient 506, such as a smart watch. For example, a Parkinson's patient may be fitted with a wearable sensor that measures tremors, such as by measuring the frequency and amplitude of such tremors. U.S. Patent Application Serial No. 17/137,110, filed December 29, 2020, discusses determining which symptoms and/or side effects are most sensible to score for a given patient when the stimulation parameters are optimized.

The clinical responses, either reported by the patient or measured by a sensor, may be converted to clinical response values 508, also referred to as clinical response scores. In an example, the clinical response values 508 may be computed based on the intensity, frequency, or duration of one or more of tremor, rigidity, or bradykinesia responses. Based upon the received clinical response values 508, the feedback control logic 501 can adjust electrode configurations or values of one or more stimulation parameters 503. The feedback control logic 501 can send the adjusted (new or revised) stimulation setting 504, such as the electrode configuration or the adjusted stimulation parameter values, to further configure the DBS controller 505 to change the stimulation parameters of the leads implanted in patient 506 to the adjusted values.

The feedback-control loop as illustrated in FIG. 5 can continue until an optimal, desired, or acceptable outcome is reached, such as maximizing therapeutic effectiveness while minimizing unwanted side effects, or until a specific stop condition is reached such as number of iterations, time spent in programming session, or the like. An outcome may be considered optimal, desired, or acceptable if it meets certain threshold values or tests (e.g., improved clinical response for the patient, faster programming of the device, increased battery life, and/or control multiple independent current sources and directional lead). Such an iterative process of looking for a stimulation setting (e.g., an electrode configuration and stimulation parameter values for the electrode) is referred to as a stimulation setting optimization process. The outcome being reached is referred to as an optimization criterion, and the resultant stimulation setting is referred to as an optimal base stimulation setting (BSS). By way of example and not limitation, the optimization criterion may include possible optimal clinical outcome within the parameters chosen; time spent, iterations taken, or power usage to explore the search space until a desired clinical outcome is reached (assuming multiple outcomes with the same or comparable clinical response); among others.

In an example, the optimization criterion includes the clinical response values 508 exceeding a threshold value or falling into a specified value range, indicating a satisfactory therapeutic outcome has reached. Depending on how the clinical response values are computed, one or more optimal base stimulation settings may be determined. For example, the clinical response values may be computed using a single response effect (e.g., one of bradykinesia, tremor, or rigidity). Accordingly, three optimal base stimulation settings may be generated: a first optimal base stimulation setting (BSS₁) corresponding to a bradykinesia score exceeding a threshold, a second optimal base stimulation setting (BSS₂) corresponding to a tremor score exceeding a threshold, and a third optimal base stimulation setting (BSS₃) corresponding to a rigidity score exceeding a threshold. In another example, the clinical response values can be a composite score computed as a weighted combination of multiple clinical effects, such as a%* bradykinesia + b%* tremor + c%* rigidity. Accordingly, a fourth optimal base stimulation setting (BSS₄) can be generated, corresponding to the composite clinical response score exceeding a threshold. In some examples, the stimulation setting optimization can be performed in an in-clinic programming session such during implantation or revision of a DBS system or device follow-up, where a clinician may use a graphical user interface (GUI) interacting with the feedback control logic 501 to generate one or more optimal base stimulation settings, as discussed below with reference to FIG. 6A.

The optimal base stimulation settings (e.g., BSS₁ through BSS₄), may be stored in the memory 404. In an example, a stimulation setting, along with the corresponding unique clinical response indicator (e.g., weighted combination of clinical effects with unique weight factors) form a stimulation program 514, which can also be stored in the memory 404. Each stimulation programmed can be associated with, or tagged by, one or more unique clinical response indicators.

In some examples, the clinical response values 508 may be weighted according to the time at which the test took place. It has been found that, for at least some patients or at least some testing procedures, patient fatigue occurs over time which may degrade or otherwise change the clinical response values (whether obtained from the patient, clinician, or a sensor). For example, more recent clinical response values 508 may be weighted higher than earlier clinical response values as the more recent clinical response values are more predictive of the clinical response to the adjusted stimulation parameters because these later tests have a similar amount of patient fatigue. By way of example and not limitation, the weights can be in a range from 1.05 to 1.5.

In various examples, the stimulation parameter control system 500 may be executed on its own and is not connected to a controller. In such instances it may be used to merely determine and suggest programming parameters, visualize a parameter space, test potential parameters, etc.

The process of iterative search for a stimulation setting (e.g., an electrode configuration and/or stimulation parameter values) typically involves significant computation and time, especially when electrode configuration involves segmented electrodes in a directional lead (e.g., lead 219). If testing all possible settings in the entire parameter space (including electrode configurations and combinations of stimulation parameter values) is done as comprehensively as possible, stimulation would need to be provided to the patient for each possible setting, which may end up with a burdensome and time-consuming programming session. Because practically a programming session may only last a few hours, only a fraction of possible electrode configuration and stimulation parameter combinations can reasonably be tested and evaluated. To reduce the time taken and to improve the efficiency of stimulation setting optimization process, a reduced or restricted electrode configuration and parameter search space can be used. By applying limitations or constraints to the electrode configurations and parameter values, the restricted search space can include a subset of electrodes (e.g., a subset of ring electrodes and/or a subset of segmented electrodes on a lead) that are selected as active electrodes for delivering stimulation, and values or value ranges for one or more stimulation parameters (e.g., a range of current amplitude ranges for an active electrode). Stimulation setting optimization, when performed within such a search space, can be more efficient and cost-effective than searching through the entire parameter space for one or more optimal base stimulation settings such as BSS₁ - BSS₄ as discussed above.

The search space identifier 510 can automatically determine a search space 512 for a stimulation lead (e.g., leads 218 or 219) at a neural target, such as a region in a brain hemisphere, by imposing certain limitations or constraints on the electrode configurations and/or parameter values or value ranges. In an example, the search space 512 can be determined based on spatial information of the lead, such as lead positions with respect to neural targets, which can be obtained from imaging data of the lead and patient anatomy. Additionally or alternatively, the search space 512 can be determined based on physiological information such as physiological signals sensed by the electrodes at their respective tissue contact locations. The physiological information may include patient clinical responses to stimulation. In some examples, prior knowledge about patient medical condition, health status, DBS treatment history may also be utilized to determine the search space 512. In an example, the search space identifier 510 may exclude those electrodes on the lead that are out of a region of interest, such that the search space includes only those electrodes within the target of interest. One or more stimulation parameters may be restricted to take certain values or within value ranges. For example, the restricted search space may include certain electrode positions and value ranges for stimulation current amplitude, frequency, or pulse width. The feedback control logic 501 can determine one or more optimal base stimulation settings (e.g., BSS₁ - BSS₄) by searching through the identified search space 512. The identified search space 512 can be stored in the memory 404. Examples of determining the search space 512 and adjusting electrode configuration and/or parameter values within the search space 512 are discussed below, such as with reference to FIG. 6B.

The feedback control logic 501 may include a machine learning engine 502 that can facilitate the stimulation parameter control system 500 (or a user of the system) to explore the search space in order to choose values for programming the DBS controller 505. The machine learning engine 502 can employ supervised or unsupervised learning algorithms to train a prediction model, and use the trained prediction model to predict patient clinical responses to an untested stimulation setting (e.g., untested stimulation parameter values or untested electrode configurations), or to estimate or predict stimulation parameters values or electrode configurations that, when provided to the DBS controller 505 to deliver stimulation accordingly to the patient 506, would produce desired or improved clinical responses. Examples of the learning algorithms include, for example, Naive Bayes classifiers, support vector machines (SVMs), ensemble classifiers, neural networks, Kalman filters, regression analyzers, etc. The machine learning engine 502 can build and train a prediction model using training data, such as stimulation parameter values and corresponding patient clinical responses. The training date can be acquired from a training session such as performed in a clinic. Additionally or alternatively, the training data can be obtained from historical data acquired by the stimulation parameter control system 500. With its learning and prediction capability, the machine learning engine 502 can aid a user (e.g., a clinician) in exploring the stimulation parameter space more effectively and more efficiently to produce results that are optimal, desired, or acceptable.

In some examples, the machine learning engine 502 can utilize imaging data to inform the choice of the next set of values. This will be particularly useful, when the algorithm finds itself in a region of parameter space for which the clinical responses are not substantially affected by the changes in the stimulation parameters, and the choice of next step is not apparent from the patient response alone. Imaging data that provides information about the location of the lead in the patient's brain along with priors informing the algorithm of which directions may be better choices for the next step could lead to faster convergence.

In some examples, the machine learning engine 502 can determine expected outcomes for parameter values that have not yet been tested based upon what the machine learning engine 502 has "learned" thus far, and provide a recommendation for a next set of values to test. Here, testing refers to the iterative testing required to find an optimal stimulation setting for configuring the DBS controller 505. The recommendation for a next set of values to test is based upon which of the determined expected outcomes meet a set of designated (determined, selected, preselected, etc.) criteria (e.g., rules, heuristics, factors, and the like). For example, rules considered may include such factors as: the next set of values cannot be one of the last 10 settings tested or cannot be too close to previously tested setting. Accordingly, the feedback control logic 501 with its machine learning engine 502 is used to systematically explore the stimulation parameter space based upon what it has learned thus far and (optionally) different rules and/or heuristics that contribute to achieving optimal outcomes more efficiently.

The process for determining expected outcomes for parameter values that have not yet been tested may involve use of other data for machine learning. For example, data from other programming sessions for the same patient as well as from other patients may be used to train the machine learning engine 502. In some examples, no prior data may be used. In this case, the machine learning engine 502 may use data learned from this patient only in one particular setting. In other examples, data from the same patient but from previous sessions may be used. In some examples all patient data from all sessions may be used. In some examples all patient data utilizing lead location information (knowledge of lead location in space relative to anatomy) may be used. Different other combinations are also possible.

In order to use this data for machine learning purposes, the data may first be cleansed, optionally transformed, and then modeled. In some examples, new variables are derived, such as for use with directional leads, including central point of stimulation, maximum radius, spread of stimulation field, or the like. Data cleansing and transformation techniques such as missing data imputation and dimension reduction may be employed to prepare the data for modeling.

The machine learning engine 502 may determine how best a predicted outcome meets the optimal outcome metrics. Various optimization techniques may be used, examples of which may include but are not limited to: optimization algorithms and estimation procedures used to fit the model to the data (e.g., gradient descent, Kalman filter, Markov chain, Monte Carlo, and the like); optimization algorithms reformulated for search (e.g., simulated annealing); spatial interpolation (e.g., kriging, inverse distance weighting, natural neighbor, etc.); supplementary methods that aid the optimization process (e.g., variable selections, regularization, cross validation, etc.); other search algorithms (e.g., golden-section search, binary search, etc.). Using any of these techniques, the machine learning engine 502 can decide whether a particular predicted outcome for a set of stimulation parameter values is the fastest sufficing outcome, the best possible clinical outcome, or the optimal outcome with least battery usage, for example.

The feedback control logic 501 may be used to search and configure different types of stimulation parameters of the various leads potentially causing different clinical effects upon the patient 506. Examples of the stimulation parameters may include electrode configurations (electrode selection, polarities, monopolar or bipolar modes of stimulation), current fractionalization, current amplitude, pulse width, frequency, among others. Given these possible stimulation parameters, the stimulation parameter control system 500 can move about the parameter space in different orders, by different increments, and limited to specific ranges. In some examples, the stimulation parameter control system 500 may allow the user to provide search range limitations to one or more of the stimulation parameters to limit the range for that stimulation parameter over which the system will search for parameters. For example, the user may restrict which electrodes can be used for stimulation or may restrict the amplitude or pulse width to a certain range or with a selected maximum or minimum. As one illustration, based on the site of implantation, the user may be aware that the distal-most and proximal-most electrodes are unlikely to produce suitable stimulation and the user limits the range of electrodes to exclude these two electrodes.

For a lead with segmented electrodes, the number of possibilities for parameter selection can be very large when combinations of electrodes and different amplitudes on each electrode are possible. In some examples using a lead with segmented electrodes, the selection of electrodes used for stimulation may be limited to fully directional selections (i.e., selection of only a single segmented electrode) and fully concentric selections (i.e., all electrodes in a single set of segmented electrodes are active with the same amplitude). In other examples, the initial movement through parameter space may be limited to fully directional and fully concentric selections. After a set of stimulation parameters is identified using these limits, variation in the selection of electrodes may be opened up to other possibilities near the selection in the identified set of stimulation parameters to further optimize the stimulation parameters.

In some examples, the number of stimulation parameters that are varied and the range of those variations may be limited. For example, some stimulation parameters (e.g., electrode selection, amplitude, and pulse width) may have larger effects when varied than other stimulation parameters (e.g., pulse shape or pulse duration). The movement through stimulation parameter space may be limited to those stimulation parameters which exhibit larger effects. In some examples, as the stimulation parameter control system 500 proceeds through testing of sets of stimulation parameters, the system may observe which stimulation parameters provide larger effects when varied and focus on exploring variation in those stimulation parameters.

In some examples, the stimulation parameter control system 500 can include a user interface for visualizing exploration of the stimulation parameter space as the system determines new and better parameter values to test until a solution is determined that fits within certain designated thresholds or a stop condition is reached. In some examples of the stimulation parameter control system 500, the user interface is part of the feedback control logic 501. In other examples, the user interface may be part of another computing system that is part of the stimulation parameter control system 500 or may be remote and communicatively connected to the stimulation parameter control system 500. The user interface may present to a user (such as a clinician, physician, programmer, etc.) a visualization of the predicted expected outcomes for (some of) the stimulation parameter values not yet tested and a recommendation for the next set of stimulation parameter values to test.

In some examples where a deep brain stimulator is configured via the DBS controller 505 with at least one set of stimulation parameter values forwarded by the feedback control logic 501, the clinician may monitor the patient throughout the process and record clinical observables in addition to the patient 506 being able to report side effects. When a side effect is observed, the various search algorithms will take that fact into account when selecting/suggesting a next set of values to test. In some examples, for example, those that select contacts via monopolar review, other parameters may be changed until they cause a side effect, which case is noted as a boundary. For example, in monopolar review where amplitude is another stimulation parameter being varied, the amplitude may be increased progressively until a side-effect is observed.

In some examples, more than one clinical metric (e.g., tremor, rigidity, bradykinesia, etc.) may be important observables. Different examples of the stimulation parameter control system 500 may handle these metrics differently. For example, some examples might identify an ideal location for each metric and choose one ideal location between them, set in the patient's remote controller so the patient can choose as needed, or chose a best combined outcome. As another example, some examples may search multiple outcomes at the same time and use the best combined score as the best outcome or find a best location for each metric individually. As yet another example, some examples may use a sequential process for selecting stimulation parameter values for multiple outcomes. For example, a system may search parameter space for a first outcome (e.g., bradykinesia) and, upon finding a suitable end condition, then search parameter space for a second outcome (e.g., rigidity). While searching parameter space for the first outcome, clinical response values for both the first and second outcomes can be obtained. Thus, when the system switches to the second outcome there are already a number of clinical response values for that outcome which will likely reduce the length of the search.

In some examples, two stimulation leads may be implanted to produce stimulation effects on two sides of the body (e.g., the right and left sides of the body). The same procedure described herein can be used to either jointly determine the stimulation parameters for the two leads by exploring the joint parameter space or individually determine stimulation parameters for the two leads by exploring the parameter space for each lead individually. In some examples, the user may determine for each side of the body which clinical response is dominant or most responsive. This may be done, for example, by having the patient perform a single task which captures multiple responses (e.g. connecting dots on the screen to monitor tremor and bradykinesia of the movement) or a small series of tasks. This enables the system to determine which clinical response to use to identify the stimulation parameters for that side of the body.

As noted, the feedback may be provided directly by the patient 506, entered by an observer such as a clinician (not shown), or may be provided by means of a sensor 507 associated with and in physical, auditory, or visual contact with the patient 506. In an example, the sensor 507 may be included in a wearable device associated with patient 506, such as a smart watch. In an example where the feedback can be monitored automatically or semi-automatically, such as with use of sensor 507, it may not be necessary for a clinician or other observer to be present to operate the stimulation parameter control system 500. Accordingly, in such examples a user interface may not be present in system 500.

In some examples, the stimulation parameter control system 500 may determine one or more optimal base stimulation settings using predicted clinical responses for untested stimulation parameter values or untested electrode configurations without actually delivering stimulation. Such base stimulation settings are referred to as "estimated" base stimulation settings, to distinguish from the "tested" base stimulation settings (e.g., BSS₁ - BSS₄) that are based on the tested clinical response (either reported by the patient or measured by a sensor) to actually delivered stimulation. For examples, based on the "tested" base stimulation settings BSS₁ - BSS₄, the stimulation parameter control system 500 may estimate an optimal base stimulation setting associated with a composite clinical response defined as x%* bradykinesia + y%* tremor + z%* rigidity, or simply denoted by the weight factors (x%, y%, z%). By way of example and not limitation, the stimulation parameter control system 500 may generate a fifth optimal base stimulation setting (BSS₅) corresponding to a composite clinical response using bradykinesia and tremor only, each weighted 50%; a sixth optimal base stimulation setting (BSS₆) corresponding to a composite clinical response using tremor and rigidity only, each weighted 50%; a seventh optimal base stimulation setting (BSS₇) corresponding to a composite clinical response using bradykinesia and rigidity only, each weighted 50%; or an eighth optimal base stimulation setting (BSS₈) corresponding to a composite clinical response using bradykinesia, tremor, and rigidity weighted 40%, 40%, and 20%, respectively. Similar to the "tested" base stimulation settings BSS₁ - BSS₄, the "estimated" base stimulation settings BSS₅ - BSS₈, associated with their respective clinical response indicators (e.g., weight factors for clinical effects), can be stored in the memory 404 as respective stimulation programs 514. In an example, the stimulation programs 514 may be stored in a lookup table, as shown in Table 1, in which each tested or estimated base stimulation setting (e.g., BSS₁ through BSS₈) is tagged by respective clinical response indicators or weight factors for clinical effects. In an example, the memory 404 can be a part of memory circuitry internal to the IPG (e.g., IPG 127 or IPG 210). The RC 128 or the CP 129 can request access to the memory 404 to retrieve therefrom one or more stored stimulation programs 514 or the search space 512.

**Table 1. Tested (⁺) and Estimated (^{*}) Stimulation Programs**

| Stimulation Program # | Weight factors for clinical effects (%Bradykinesia, %Tremor, % Rigidity) | Base Stimulation Setting |
|---|---|---|
| 1⁺ | (100, 0, 0) | BSS₁ |
| 2⁺ | (0, 100, 0) | BSS₂ |
| 3⁺ | (0, 0, 100) | BSS₃ |
| 4⁺ | (50, 25, 25) | BSS₄ |
| 5^{*} | (50, 50, 0) | BSS₅ |
| 6^{*} | (0, 50, 50) | BSS₆ |
| 7^{*} | (50, 0, 50) | BSS₇ |
| 8^{*} | (40, 40, 20) | BSS₈ |

FIG. 6A illustrates, by way of example and not limitation, a graphical user interface (GUI) 600 operable on an external device such as the clinician program (CP) 129, which allows a user (e.g., a clinician) to program a stimulation setting into the IPG (e.g., IPG 127 or IPG 210). The GUI 600 can be rendered on a display of the CP 129, and provide a clinician with a visual indication of how a stimulation setting (e.g., electrode configuration and/or values for a plurality of stimulation parameters) may interact with target tissue (e.g., brain tissue in the context of DBS) in which the electrodes are implanted. The GUI 600 may be used during surgical implantation of the leads 218 or 219 and the IPG 210, but may also be used after implantation to assist in creating, modifying, or selecting a therapeutically useful stimulation program for the patient.

The GUI 600 can be controlled by a cursor 601 that the user can move using a tracking device such as a mouse connected to the CP 129. The GUI 600 may include a stimulation waveform interface 604 that allows a user to select, and adjust a value of, a stimulation waveform parameter, such as a stimulation amplitude (e.g., a current I), a frequency (F), or a pulse width (PW) of stimulation pulses. In some examples, the waveform interface 604 can be significantly more complicated, particularly if the IPG 210 supports the provision of stimulation that is more complicated than a repeating sequence of pulses. In some examples, the waveform interface 604 may allow a user to select biphasic or monophasic pulses, and to select whether passive charge recovery will be used (not shown).

The GUI 600 may include an electrode configuration interface 605 which allows the user to select and modify a particular electrode configuration, such as specifying which electrodes are active electrodes (ON) to provide stimulation, and which electrodes are inactive electrodes (OFF) to refrain from providing stimulation. For an active electrode, the user may also use the electrode configuration interface 605 to specific polarity and relative magnitude for that active electrode. As illustrated in FIG. 6A, the user may use the electrode configuration interface 605 to designate an electrode as an anode (A), a cathode (C), or an inactive electrode (OFF), and to specific an amount of the total anodic or cathodic current +I or -I (specified in the waveform interface 604) that a selected active electrode will receive in a form of fraction or percentage (X%) of the total current provided. Such a split of current among multiple electrodes is referred to as current fractionalization. In an example as illustrated in FIG. 6A, the case electrode 612 (Ec) is specified as the only anode that receives 100% of anodic current +I. The corresponding cathodic current -I is split among a set of selected active cathode electrodes including E2 (18% of -I), E4 (52% of -I), E5 (8% of -I), and E7 (22% of -I). One or more electrodes can be chosen to act as anodes or cathodes at a given time, allowing the electric field in the tissue to be shaped. Once the stimulation waveform interface 604 and electrode configuration interface 605 are determined, they can be sent to the IPG 210, which can produce stimulation pulses accordingly, and deliver the stimulation pulses to the patient.

The GUI 600 can include a leads interface 602 showing an image 603 of the lead being used for the patient. By way of example and not limitation, the lead shown in the image 603 includes two ring electrodes E1 and E8, a first group of segmented electrodes E2-E4 about a circumference of a first longitudinal position of the lead, and a second group of segmented electrodes E5-E7 about a circumference of a second longitudinal position of the lead. In this example, segmented electrodes E2, E4, E5, and E7 are selected as cathodes to receive fractionalized current. Such current fractionalization sets a particular position for a cathode pole 619 in a three-dimensional space. The position of this cathode pole 619 can be quantified at a particular longitudinal position L along the lead (e.g., relative to a point on the lead such as the longitudinal position of electrode E1). If a direction lead such as lead 219 is uses, the position of the cathode pole 619 may further be quantified at a particular rotational angle θ (e.g., relative to a particular angle on the lead such as relative to the center of electrode E2). The position (L, θ) of the cathode pole 619, as shown in the leads interface 602, may be virtual; that is, the position may not necessarily be at a physical position of any electrode on the lead, but a point in the three-dimensional space of the leads interface 602. In some examples, the interface 602 can include a selection to access a library of images 603 of the types of leads (e.g., 218 or 219) that may be implanted in different patients, which may be stored with the CP 129. The cursor 601 can be used to select an electrode such as any of E1-E8, or the case electrode Ec, or a pole such as cathode pole 619.

The CP 129 can include and execute an electrode configuration algorithm to determine a position of the cathode pole 619 in the three-dimensional space from a given electrode configuration, or determine an electrode configuration from a given position of the cathode pole 619. For example, the user can place the position of the cathode pole 619 using the cursor 601. The electrode configuration algorithm can then be used to compute an electrode configuration (e.g., current fractionalization across a plurality of selected active electrodes) that best places the cathode pole 619 in this position. The electrode configuration algorithm may thus calculate that electrode E4 should receive the largest share of cathodic current (52%*-I), while E2, E7, and E6 which are farther away from the cathode pole 619 receive lesser percentages, as shown in the stimulation parameters interface 604. By involving more than one electrode, cathode pole 619 is formed as a virtual pole not as the position of any of the physical electrodes. Again, the electrode configuration algorithm can also operate in reverse: from a given electrode configuration, the position of the cathode pole 619 can be determined. The electrode configuration algorithm is described further in U.S. Patent Application Publication 2019/0175915.

The GUI 600 can further include a visualization interface 606 that allows a user to view a stimulation field image 622 formed on a lead given the selected stimulation parameters and electrode configuration. The stimulation field image 622 is formed by field modelling in the CP 129. The visualization interface 606 may include tissue imaging information, such as imaging information of different brain tissue structures 624A, 624B and 624C in the context of DBS. Such tissue imaging information may come from a Magnetic Resonance Image (MRI) or Computed Tomography (CT) image of the patient, may come from a generic library of images, and may include user defined regions. The GUI 600 can overlay the lead image 621 and the stimulation field image 622 with the tissue imaging information in the visualization interface 606 so that the position of the stimulation field image 622 relative to the various tissue structures 624A-624C can be visualized. The various images shown in the visualization interface 606 (i.e., the lead image 621, the stimulation field image 622, and the tissue structures 624A-624C) can be three-dimensional in nature, and hence may be rendered to allow such three-dimensionality to be better appreciated by the user, such as by shading or coloring the images, etc. A view adjustment interface 607 may allow the user to use the cursor 601 to move or rotate the images. As illustrated in FIG. 6A, a cross-section interface 608 allows the various images to be seen in a particular two-dimensional cross section, such as a cross section 609 taken perpendicularly to the lead image 621 and through the segmented electrodes E2, E3, and E4.

The GUI 600 can be particularly useful because it allows the electric field as reflected in stimulation field image 622 to be seen relative to surrounding tissue structures 624A-624C. This allows the user to adjust the stimulation parameters to recruit, or avoid recruiting, particular tissue structures. Assume for example that it is desirable for a given patient to stimulate tissue structure 624A, but to not stimulate tissue structures 624B or 624C where the stimulation may cause undesired side effects. The clinician can then use the GUI 600 to adjust stimulation (e.g., to adjust the stimulation parameters or the electrode configuration) to move the stimulation field (e.g., the cathode pole 619) to a proper position (L, θ). In the example shown, and as best seen in the cross-section interface 608, higher cathodic currents are provided at electrodes E4 (52%*-1) and E2 (18%*-I), because these electrodes are generally facing towards tissue structure 624A where stimulation is ideally applied. By contrast, electrode E3 carries no cathodic current because it generally faces towards tissue structure 624B where stimulation is ideally avoided. The result is a stimulation field that is more predominant in tissue structure 624A and less predominant in tissue structure 624B, as shown in the visualization interface 606.

Especially in a DBS application, it is important that correct stimulation parameters be determined for a given patient. Improper stimulation parameters may not yield effective relief of a patient's symptoms, or may cause unwanted side effects. To determine proper stimulation, a clinician may use the GUI 600 to try different combinations of stimulation parameters and electrode configurations. This may occur, during a DBS patient's surgery when the leads are being implanted, or during a post-surgery office visit after the patient has had a chance to heal and after the position of the leads stabilize in the patient. During an in-clinic programming session, a clinician may use the GUI 600 that interacts with the feedback control logic 501 to generate one or more optimal base stimulation settings, as discussed above with reference to FIG. 5.

FIG. 6B illustrates, by way of example and not limitation, a diagram 650 of an electrode configuration and parameter search space, such as one determined by the search space identifier 510. The search space defines a sub-space of electrode configurations and stimulation parameter values that may be used during the stimulation setting optimization to search for one or more optimal base stimulation settings. As discussed above, the search space may be identified for a lead with known electrodes types and locations on the lead (e.g., non-directional lead 218 with ring electrodes, or directional lead 219 with segmented electrodes) positioned at a target stimulation region such as a region in a brain hemisphere. In the example shown in FIG. 6B, the electrode configurations are represented by the electrode positions on the lead (e.g., vertical locations of ring electrodes), the stimulation parameters are represented by stimulation current amplitude, and the search space is represented by a heatmap that depicts intensities of clinical responses (represented by grayscale or colormap) to stimulations delivered over a range of electrode positions, such as vertical locations relative to the leads geometry (y-axis) and over a range of stimulation parameter values such as current amplitudes (x-axis). Intensities of clinical responses may be represented by clinical response scores computed using, for example, a weighted combination of one or more clinical effects (e.g., bradykinesia, tremor, or rigidity). Clinical effects may be evaluated for a plurality of electrodes at different locations and provided with respective stimulation current amplitudes, such as in an in-clinic programming session. The actually tested clinical effects (as outcomes of actually delivered electrostimulation) may be plotted as data points on the diagram 650. For example, data point 651 represents positive effects, such as determined based on the clinical response score exceeding a threshold indicating therapeutic efficacy and no or tolerable side effects. Data points 652 represents negative effects, such as determined based on the clinical response score falling below a threshold indicating less therapeutic efficacy and intolerable side effects.

In addition to the actually tested clinical effects (as outcomes of actually delivered electrostimulation) such as through an in-clinic programming session, clinical effects may be predicted for untested electrode configurations and untested stimulation parameter values without actually delivering electrostimulation and evaluating the resultant clinical responses. The prediction can be carried out using the machine learning engine 502 of the feedback control logic 501 as discussed above with reference to FIG. 5. The predicted clinical effects may be depicted as data points on the diagram 650. Similar to the actually tested clinical effects, the predicted clinical effects can include positive effects and negative effects, represented by respective data points on the diagram 650. As illustrated in FIG. 6B, the diagram 650 may include a positive-effect region 661 covering substantially data points representing actually tested and predicted positive effects, and a negative-effect region 662 covering substantially data points representing actually tested and predicted negative effects. The positive-effect region 661 defines a search space from which stimulation parameters and electrode configurations may be selected for an electrostimulation therapy without causing undesirable side effects, while the negative-effect region 662 defines stimulation parameters and electrode configurations that need to be avoided in an electrostimulation therapy to prevent undesirable side effects.

A separation line or curve 671 that separates the positive-effect region 661 from the negative-effect region 662 may be determined using, for example, a least-square method. In some examples, the predicted clinical effects may each be associated with a probability of the clinical response being a positive effect or a probability of the clinical response being a negative effect. Accordingly, the negative-effect region 662 and the positive-effect region 661 may also be associated with respective probabilities. As illustrated in FIG. 6B, the separation line or curve 671 separates the positive-effect region 661 from the negative-effect region 662 that has a high probability of causing immediate side effects (e.g., side effects that would occur within 30 seconds of stimulation). A different separate line or curve 672 separates the positive-effect region 661 from the negative-effect region 662 that has a lower probability of causing immediate side effects, or more likely to cause long-term side effects observable with longer stimulation (e.g., over 30 minutes or one hour of stimulation) or when stimulation is delivered to multiple electrodes simultaneously. Also shown on the diagram 650 is another negative-effect region 663 corresponding to electrode locations where the stimulation provided therein are estimated to lead to certain side effects, such as mood-related side effects that typically take a longer time to appear. As the negative-effect region 663 extends through a wide stimulation current amplitude range, these electrode locations are to be excluded from the search space, that is, they can be designated as inactive electrodes (OFF) to refrain from providing stimulation.

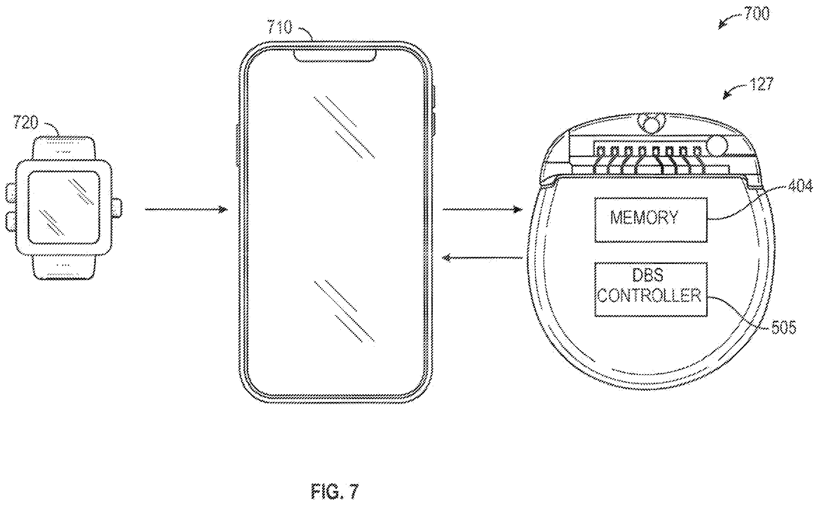

The diagram 650 may be displayed to a user, such as on the GUI 600 of the CP 129, where a user (e.g., a clinician or the patient) may select active electrodes or adjust stimulation parameter values within the "positive-effect" region 661. For example, the user may only be allowed to select stimulation current amplitude for an electrode at a particular location to be within a specific amplitude range. In some examples, information about the base stimulation settings, including the tested or estimated optimal base settings such as BSS₁ - BSS₈ as shown in Table 1, may be displayed on the diagram 650. FIG. 6B illustrates eight base stimulation programs graphically represented by arrows 680, each comprising electrode location information (y-axis) and stimulation current values (x axis). In some examples, the diagram 650 may be displayed on a user interface of the RC 128, such as a mobile device (e.g., a smartphone or a tablet) operable by the patient for in-home titration of an base stimulation program, as to be discussed in the following with reference to FIG. 7.

As discussed above with reference to FIG. 5, in some examples, the feedback control logic 501 may be implemented in a CP 129 or in a RC 128. The CP 129 may be used in a clinical setting during IPG implantation or post-surgery office visit to generate, and store in the IPG, one or more optimal base stimulation settings (e.g., BSS₁ - BSS₈ as shown in Table 1) in an in-clinic programming session. The base stimulation settings thus generated, even though deemed optimal at the time of programming (e.g., one or more clinical responses captured during the in-clinic programming session satisfy a specific criterion), may nevertheless produce sub-optimal patient outcome such as due to changes in patient state (e.g., progression of an existing health condition or development of new health condition, or subsequent to treatment) monthly, weekly, daily, or even hourly after the in-clinic programming session. Re-optimization at the clinic may not be practically or financially feasible for some patients.

To address this concern, the present inventors have proposed techniques for remotely controlled in-home titration of base stimulation settings such as generated in an in-clinic programming session. FIG. 7 illustrates an example of an in-home therapy titration system 700 for modifying a pre-generated stimulation program for DBS. The in-home titration session may be performed by a user (e.g., the patient) with the assistance of a remotely controlled therapy titration device 710. In an example, the remotely controlled therapy titration can be a software application ("App") implemented in a smartphone, a tablet, or other mobile devices. The therapy titration device 710, which is an example of the RC 128, may include an Application Programming Interface (API) that allows the user to request and retrieve from the IPG 127 one or more base stimulation programs 514 and the search space 512 stored in the memory 404, including the base stimulation program presently being used to provide DBS therapy. For example, if the IPG 127 is presently programmed with Program 4 in Table 1, the therapy titration device 710 may acquire clinical response information reported by the patient or automatically measured by a sensor in a wearable device 720. The clinical response information indicates therapeutic effects and/or side effects of the base stimulation program. Due to changes in patient state, the acquired clinical responses may be different from the optimal or desired clinical responses observed during the course of in-clinic testing even though the same stimulation program (e.g., Program 4) is used to provide DBS therapy. The therapy titration device 710 may identify clinical effects (e.g., one or more of tremor, rigidity, or bradykinesia) that have worsened from what was recorded in the past during the in-clinic testing session, and prioritize or boost the identified worsened clinical effects, such as by increasing the weights for the worsened clinical effects, and/or decreasing the weights for other clinical effects that have not worsened from the in-clinic testing session. For example, if the presently acquired or measured clinical responses under Program 4 demonstrate that the tremor response has worsened from the past tremor response observed during in-clinic testing, then the therapy titration device 710 may increase the weight for the tremor response, such as from its previous weight 25% to a new weight 40%. The therapy titration device 710 may additionally or alternatively decrease the weight for one or more other clinical effects, such as rigidity or bradykinesia. The therapy titration device 710 may implement the feedback control logic 501 as discussed above with reference to FIG. 5, and modify the present base stimulation program (e.g., Program 4) by modifying electrode configuration (e.g., selecting different electrodes) and/or adjusting values of one or more stimulation parameters within the stored search space 512, while evaluating a composite clinical response score defined with the new weights (e.g., Tremor response given a weight of 40%). In an example, the therapy titration device 710 may display on a GUI a graph of the search space such as the diagram 650 illustrated in FIG. 6B, and the patient may search through the search space (e.g., the positive-effect region 661) until a desired outcome is achieved, such as the clinical responses achieving a maximal or a desired amount of improvement in clinical effect. In some examples, searching the search space for an optimal stimulation setting can be carried out using the iterative optimization as discussed above with respect to the feedback control logic 501.

The DBS controller 505 may program the IPG with the resultant modified stimulation program (e.g., Program 4'), replacing the existing base stimulation (e.g., Program 4). The modified stimulation program may be stored in the memory 404. In an example, the modified stimulation program may be stored in a new patient titrated program slot, which can be a dedicated memory space other than the space for storing the base stimulation programs. Alternatively, the base stimulation program in the memory may be overwritten by the modified stimulation program.

In some examples, based on the new weights assigned to the identified worsened clinical effects, the therapy titration device 710 may select one of the stored base stimulation programs stored in the memory 404 to replace the existing base stimulation program used by the IPG. In an example, the therapy titration device 710 may select, from the base stimulation programs stored in the memory 404, a base stimulation program associated with weights for clinical effects (for computing a clinical response score) that best match the new weights assigned to the identified worsened clinical effects. In an above example where the weight for tremor response is increased to 40%, the base stimulation Program 8 has a similar weight for tremor response. As such, the therapy titration device 710 may start with the stimulation setting in the base Program 8, and perform iterative optimization as discussed above to find a modified stimulation setting.

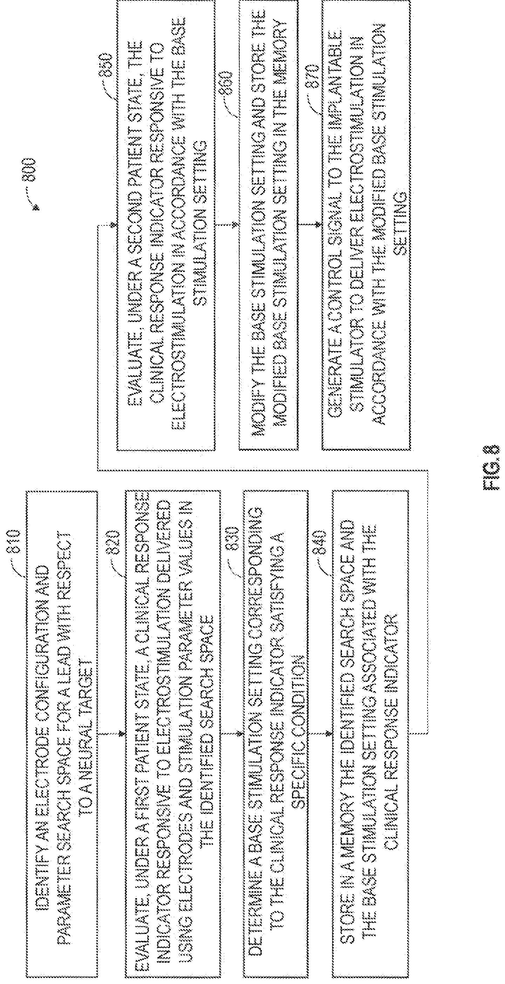

FIG. 8 is a flow chart illustrating, by way of example and not limitation, a method 800 for creating base stimulation program(s) and titrating a base stimulation program in an in-home titration session and providing electrostimulation to a neural target, such as DBS to brain tissue. The method 800 may be carried out using a medical system such as the electrical stimulation system 100 in FIG. 1, or the electrical stimulation system 412 in FIG. 4. The electrostimulation may be generated by an implantable stimulator such as the IPG 127 or the IPG 210, and delivered to the neural target via a lead comprising a plurality of electrodes, such as the non-directional lead 218 or the directional lead 219. Portions of the method 800 may be implemented in an external device, such as the CP 129 or the RC 128 in FIG. 1, the computing device 400 in FIG. 4, or the therapy titration device 710 in FIG. 7.

The method 800 includes steps 810-840 for generating and storing in a memory one or more base stimulation settings as well as a search space of electrode configurations and parameter values (also referred to as a "search space"), which can be performed in an in-clinic programming session under a first patient condition; and steps 850-870 for a remote-controlled therapy optimization, which can be performed in an in-home therapy titration session under a different second patient condition, such as using the therapy titration device 710. At 810, a search space for the lead with respect to the neural target can be identified, such as using the search space identifier 510. The search space can include a subset of the electrodes on the lead for delivering stimulation and stimulation parameter values or value ranges associated with the subset of electrodes. Identification of the search space can be based on spatial information of the lead, such as lead positions with respect to neural targets, which can be obtained from imaging data of the lead and patient anatomy. Additionally or alternatively, identification of the search space can be based on physiological information such as physiological signals sensed locally at electrode contacts. Certain limitations or constraints may be imposed on the electrode configurations and parameter values (e.g., current amplitude, frequency, or pulse width). In some examples, the search space may be graphically presented to a user (e.g., a clinician or a patient), such as a heatmap depicting intensities of clinical responses to stimulations delivered over a range of electrode positions and over a range of stimulation parameter values, as shown in FIG. 6B.

At 820, a clinical response indicator can be evaluated responsive to electrostimulation delivered using electrodes and stimulation parameter values taken from the identified search space under a first patient state. The evaluation of the clinical response indicator can be performed in an in-clinic programming session, using a programming device such as the computing device 400, the CP 129 or the RC 128. Clinical responses may be evaluated for a plurality of electrodes at different locations and injected with respective stimulation current amplitudes. The clinical response may include therapeutic benefits and/or side effects on the patient. In an example, the clinical responses may be based on patient or clinician observations. For example, motor symptoms such as bradykinesia (slowness of movement), rigidity, tremor, among other symptoms or side effects, can be scored by the patient or by the clinician upon overserving or questioning the patient. In some examples, the clinical responses can be objective in nature, such as measurements automatically or semi-automatically taken by a sensor, such as a wearable sensor associated with a mobile device. The clinical responses may be converted to a clinical response score. In an example, a clinical score may be computed based on intensity, frequency, or duration of one or more of tremor, rigidity, or bradykinesia responses.

At 830, at least one base stimulation setting may be determined based on the evaluation of the clinical response indicator. A base stimulation setting can correspond to a clinical response indicator satisfying a specific condition, such as the clinical response score falling within a specified range. A base stimulation setting, which includes an optimal electrode configuration and an optimal stimulation parameter value, can be determined using the feedback loop stimulation parameter control system 500, in which the electrode configuration or values of one or more stimulation parameters can be continuously or iteratively adjusted and the clinical response evaluated, until an optimal, desired, or acceptable outcome is reached, such as maximizing therapeutic efficacy while minimizing unwanted side effects, or until a specific stop condition is reached such as number of iterations, time spent in programming session, or the like.

Each base stimulation setting determined at 830 can be associated with a clinical response indicator. A stimulation setting in association with the clinical response indicator (e.g., a formula for computing the clinical response score) is referred to as a stimulation program. Depending on how the clinical response score is computed, one or more optimal base stimulation settings may be determined. As illustrated in Table 1, base stimulation settings created and stored in the memory may include those base stimulation settings (e.g., BSS₁ - BSS₃) associated with the clinical response scores computed based on a single response effect (e.g., bradykinesia, tremor, or rigidity), or stimulation settings (e.g., BSS₄ - BSS₈) associated with clinical response scores computed using a weighted combination of two or more clinical effects, such as a%* bradykinesia + b%* tremor + c%* rigidity.

In some examples, one or more base stimulation settings may be determined using predicted clinical responses for untested stimulation parameter values or untested electrode configurations, without actually delivering electrostimulation and evaluating the resultant clinical responses. The prediction may be carried out using a trained prediction model, such as the machine learning engine 502 of the feedback control logic 501 in FIG. 5. Such base stimulation settings generated based on the predicted clinical responses are also referred to as estimated base stimulation settings, such as base stimulation settings BSS₅ - BSS₈.

At 840, the search space identified from 810 and the at least one base stimulation setting (associated with the clinical response indicator) determined from 830 can be stored in a memory, such as a memory internal to the IPG (e.g., IPG 127 or IPG 210). Upon request, one or more base stimulation settings and/or the search space may be retrieved from the memory.

At 850, under a second patient state different from the first patient state (in which the base stimulation settings are created and stored in the memory), the clinical response indicator associated with a base stimulation setting can be re-evaluated responsive to electrostimulation delivered in accordance with the at least one base stimulation setting. Due to changes in patient state, the clinical response (or some clinical effects) may be different from the optimal or desired clinical responses observed during the course of the in-clinic programming session.

At 860, at least one base stimulation setting may be modified, such as by modifying the optimal electrode configuration or the optimal stimulation parameter value. The modification of the electrode configuration (e.g., selecting different active electrodes) or the stimulation parameter value can be done within the search space. In an example, for a base stimulation setting, the clinical response indicator presently evaluated under the second patient state can be compared with the clinical response indicator previously evaluated under the first patient state. Based on the comparison, one or more clinical effects (e.g., tremor, rigidity, or bradykinesia) that have worsened from the first patient state to the second patient state can be identified. The clinical response indicator may be modified such by increasing the weights for the worsened clinical effects, or additionally or alternatively decreasing the weights for other clinical effects that have not worsened. The base stimulation setting may be modified such as by iteratively adjusting the optimal electrode configuration or the optimal stimulation parameter value within the search space, until the modified clinical response indicator satisfies a specific condition under the second patient state. The modified base stimulation setting, in association with the modified clinical response indicator, can then be stored in the memory.

In another example, under the second patient state, clinical response indicators responsive to electrostimulation in accordance with the plurality of base stimulation settings can be evaluated. Based on a comparison between the clinical response indicators under the second patient state and the clinical response indicators under the first patient state, a base stimulation setting can be selected from the plurality of base stimulation settings. The selected base stimulation setting can then be modified, such as by modifying the electrode configuration or the stimulation parameter value of the selected base stimulation setting. The modification of the electrode configuration (e.g., selecting different active electrodes) or the stimulation parameter value can be done within the search space, until the clinical response indicator associated with the selected base stimulation setting satisfies a specific condition under the second patient state. The resultant modified selected base stimulation setting, in association with the clinical response indicator, can then be stored in the memory.

At 870, a control signal can be generated to control the implantable stimulator to deliver electrostimulation in accordance with the modified base stimulation setting.

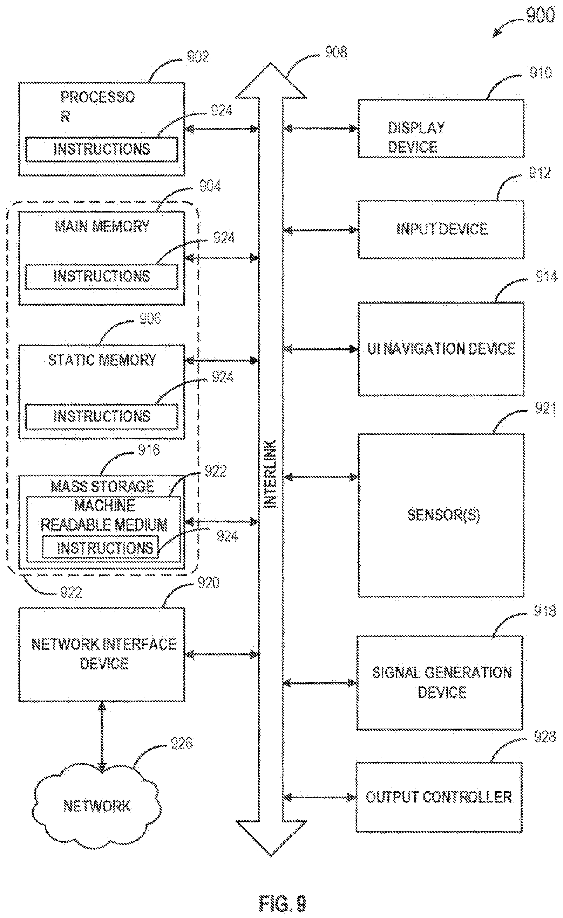

FIG. 9 illustrates generally a block diagram of an example machine 900 upon which any one or more of the techniques (e.g., methodologies) discussed herein may perform. Portions of this description may apply to the computing framework of various portions of the neuromodulation device or the external programming device.

In alternative examples, the machine 900 may operate as a standalone device or may be connected (e.g., networked) to other machines. In a networked deployment, the machine 900 may operate in the capacity of a server machine, a client machine, or both in server-client network environments. In an example, the machine 900 may act as a peer machine in peer-to-peer (P2P) (or other distributed) network environment. The machine 900 may be a personal computer (PC), a tablet PC, a set-top box (STB), a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), among other computer cluster configurations.

Examples, as described herein, may include, or may operate by, logic or a number of components, or mechanisms. Circuit sets are a collection of circuits implemented in tangible entities that include hardware (e.g., simple circuits, gates, logic, etc.). Circuit set membership may be flexible over time and underlying hardware variability. Circuit sets include members that may, alone or in combination, perform specified operations when operating. In an example, hardware of the circuit set may be immutably designed to carry out a specific operation (e.g., hardwired). In an example, the hardware of the circuit set may include variably connected physical components (e.g., execution units, transistors, simple circuits, etc.) including a computer readable medium physically modified (e.g., magnetically, electrically, moveable placement of invariant massed particles, etc.) to encode instructions of the specific operation. In connecting the physical components, the underlying electrical properties of a hardware constituent are changed, for example, from an insulator to a conductor or vice versa. The instructions enable embedded hardware (e.g., the execution units or a loading mechanism) to create members of the circuit set in hardware via the variable connections to carry out portions of the specific operation when in operation. Accordingly, the computer readable medium is communicatively coupled to the other components of the circuit set member when the device is operating. In an example, any of the physical components may be used in more than one member of more than one circuit set. For example, under operation, execution units may be used in a first circuit of a first circuit set at one point in time and reused by a second circuit in the first circuit set, or by a third circuit in a second circuit set at a different time.

Machine (e.g., computer system) 900 may include a hardware processor 902 (e.g., a central processing unit (CPU), a graphics processing unit (GPU), a hardware processor core, algorithm specific ASIC, or any combination thereof), a main memory 904 and a static memory 906, some or all of which may communicate with each other via an interlink (e.g., bus) 908. The machine 900 may further include a display unit 910 (e.g., a raster display, vector display, holographic display, etc.), an alphanumeric input device 912 (e.g., a keyboard), and a user interface (UI) navigation device 914 (e.g., a mouse). In an example, the display unit 910, input device 912 and UI navigation device 914 may be a touch screen display. The machine 900 may additionally include a storage device (e.g., drive unit) 916, a signal generation device 918 (e.g., a speaker), a network interface device 920, and one or more sensors 921, such as a global positioning system (GPS) sensor, compass, accelerometer, or other sensors. The machine 900 may include an output controller 928, such as a serial (e.g., universal serial bus (USB), parallel, or other wired or wireless (e.g., infrared (IR), near field communication (NFC), etc.) connection to communicate or control one or more peripheral devices (e.g., a printer, card reader, etc.).

The storage device 916 may include a machine readable medium 922 on which is stored one or more sets of data structures or instructions 924 (e.g., software) embodying or utilized by any one or more of the techniques or functions described herein. The instructions 924 may also reside, completely or at least partially, within the main memory 904, within static memory 906, or within the hardware processor 902 during execution thereof by the machine 900. In an example, one or any combination of the hardware processor 902, the main memory 904, the static memory 906, or the storage device 916 may constitute machine readable media.

While the machine-readable medium 922 is illustrated as a single medium, the term "machine readable medium" may include a single medium or multiple media (e.g., a centralized or distributed database, and/or associated caches and servers) configured to store the one or more instructions 924.

The term "machine readable medium" may include any medium that is capable of storing, encoding, or carrying instructions for execution by the machine 900 and that cause the machine 900 to perform any one or more of the techniques of the present disclosure, or that is capable of storing, encoding or carrying data structures used by or associated with such instructions. Nonlimiting machine-readable medium examples may include solid-state memories, and optical and magnetic media. In an example, a massed machine-readable medium comprises a machine readable medium with a plurality of particles having invariant (e.g., rest) mass. Accordingly, massed machine-readable media are not transitory propagating signals. Specific examples of massed machine-readable media may include: non-volatile memory, such as semiconductor memory devices (e.g., Electrically Programmable Read-Only Memory (EPROM), Electrically Erasable Programmable Read-Only Memory (EPSOM)) and flash memory devices; magnetic disks, such as internal hard disks and removable disks; magneto-optical disks; and CD-ROM and DVD-ROM disks.

The instructions 924 may further be transmitted or received over a communication network 926 using a transmission medium via the network interface device 920 utilizing any one of a number of transfer protocols (e.g., frame relay, internet protocol (IP), transmission control protocol (TCP), user datagram protocol (UDP), hypertext transfer protocol (HTTP), etc.). Example communication networks may include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802.11 family of standards known as WiFi^{®}, IEEE 802.16 family of standards known as WiMax^{®}), IEEE 802.15.4 family of standards, peer-to-peer (P2P) networks, among others. In an example, the network interface device 920 may include one or more physical jacks (e.g., Ethernet, coaxial, or phone jacks) or one or more antennas to connect to the communication network 926. In an example, the network interface device 920 may include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding or carrying instructions for execution by the machine 900, and includes digital or analog communications signals or other intangible medium to facilitate communication of such software.

Various examples are illustrated in the figures above. One or more features from one or more of these examples may be combined to form other examples.

The method examples described herein may be machine or computer-implemented at least in part. Some examples may include a computer-readable medium or machine-readable medium encoded with instructions operable to configure an electronic device or system to perform methods as described in the above examples. An implementation of such methods may include code, such as microcode, assembly language code, a higher-level language code, or the like. Such code may include computer readable instructions for performing various methods. The code may form portions of computer program products. Further, the code may be tangibly stored on one or more volatile or non-volatile computer-readable media during execution or at other times.

The above detailed description is intended to be illustrative, and not restrictive. The scope of the disclosure should, therefore, be determined with references to the appended claims.

## Claims

1. A system (100, 412) for providing electrostimulation to a patient, comprising:
an implantable stimulator (127, 210) configured to provide electrostimulation to a neural target of the patient via a lead (126, 218, 219) comprising a plurality of electrodes (132, 216); and
a programming device (400) communicatively coupled to the implantable stimulator (127, 210), the programming (402) device including a controller (402) configured to:
identify a search space of electrode configurations and parameter values for the lead with respect to the neural target, the search space including a subset of the plurality of electrodes for delivering stimulation and stimulation parameter values or value ranges associated with the subset of electrodes;
under a first patient state, evaluate a clinical response indicator responsive to electrostimulation delivered using electrodes and stimulation parameter values from the identified search space, and determine at least one base stimulation setting corresponding to the clinical response indicator satisfying a specific condition, the at least one base stimulation setting including an optimal electrode configuration and an optimal stimulation parameter value; and
store in a memory (404) the identified search space and the at least one base stimulation setting associated with the corresponding clinical response indicator; and
a therapy titration device (710) configured to:
under a second patient state different from the first patient state, evaluate the clinical response indicator responsive to electrostimulation in accordance with the at least one base stimulation setting;
modify the clinical response indicator based on a comparison between the clinical response indicator under the second patient state and the clinical response indicator under the first patient state;
modify the at least one base stimulation setting by iteratively adjusting the optimal electrode configuration or the optimal stimulation parameter value within the identified search space until the modified clinical response indicator satisfies a specific condition under the second patient state;
store in the memory (404) the modified at least one base stimulation setting associated with the modified clinical response indicator; and
generate a control signal to the implantable stimulator to deliver electrostimulation in accordance with the modified at least one base stimulation setting.

2. The system (100, 412) of claim 1, wherein the therapy titration device (710) is a mobile device operable by the patient and coupled to the implantable stimulator (127, 210) via a wireless link.

3. The system (100, 412) of any of claims 1-2, wherein the therapy titration device (710) includes a user interface configured to display graphically the identified search space and to receive user input to modify the at least one base stimulation setting.

4. The system (100, 412) of claim 3, wherein the user interface is configured to display graphically the identified search space as a heatmap depicting intensities of clinical responses to electrostimulation over a range of electrode positions on the lead and a range of stimulation parameter values.

5. The system (100, 412) of any of claims 1-4, wherein the controller (402) is configured to evaluate the clinical response indicator using one or more clinical effects responsive to electrostimulation delivered using electrodes and stimulation parameter values from the identified search space.

6. The system (100, 412) of claim 5, wherein:
the controller (402) is configured to evaluate the clinical response indicator using a combination of the one or more clinical effects each weighted by respective weight factors; and
the therapy titration device (710) is configured to, based on the comparison between the clinical response indicator under the second patient state and the clinical response indicator under the first patient state, identify one or more of the clinical effects that have worsened from the first patient state to the second patient state, and to modify the clinical response indicator by increasing respective weight factors for the identified one or more clinical effects.

7. The system (100, 412) of any of claims 5-6, wherein the one or more clinical effects include indicators of therapeutic efficacy or a side effect of electrostimulation.

8. The system (100, 412) of any of claims 5-7, wherein the one or more clinical effects include motor symptoms including one or more of bradykinesia, tremor, or rigidity.

9. The system (100, 412) of any of claims 5-8, wherein the therapy titration device (710) is configured to receive a user input of the one or more clinical effects.

10. The system of any of claims 5-9, wherein the therapy titration device (710) includes, or is communicatively coupled to, one or more sensors to sense the one or more clinical effects.

11. The system of any of claims 1-10, wherein the controller (402) is configured to determine, and store in the memory (404) , a plurality of base stimulation settings each associated with respective clinical response indicators, the respective clinical response indicators each evaluated under the first patient state using one or more clinical effects weighted by respective weight factors, the plurality of base stimulation settings each including respective optimal electrode configurations and respective optimal stimulation parameter values.

12. The system of claim 11, wherein the controller is configured to evaluate one or more of the plurality of clinical response indicators using weighted combinations of one or more clinical effects each weighted by respective weight factors, the weight factors associated with one of the plurality of base stimulation settings being different from the weight factors associate with another of the plurality of base stimulation settings.

13. The system of any of claims 11-12, wherein the controller is configured to:
under the first patient state, predict, using a trained machine-learning model and without delivering electrostimulation, one or more clinical effects for untested stimulation parameter values or untested electrode configurations in the identified search space;
evaluate a clinical response indicator using the predicted one or more clinical effects; and
determine an estimated base stimulation setting associated with the clinical response indicator evaluated using the predicted one or more clinical effects.

14. The system of any of claims 11-13, wherein the programming device includes a therapy titration device (710) configured to:
under a second patient state different from the first patient state, evaluate respective clinical response indicators responsive to electrostimulation in accordance with the plurality of base stimulation settings;
based on a comparison between the clinical response indicators under the second patient state and the clinical response indicators under the first patient state, select a base stimulation setting from the plurality of base stimulation settings;
modify the selected base stimulation setting by iteratively adjusting an optimal electrode configuration or an optimal stimulation parameter value of selected base stimulation setting within the identified search space until the clinical response indicator associated with the selected base stimulation setting satisfies a specific condition under the second patient state;
store in the memory (404) the modified selected base stimulation setting; and
generate a control signal to the implantable stimulator (127, 210) to deliver electrostimulation in accordance with the modified selected base stimulation setting.

## Patentansprüche

1. System (100, 412) zur Bereitstellung von Elektrostimulation für einen Patienten, aufweisend:
einen implantierbaren Stimulator (127, 210), der dazu konfiguriert ist, über eine Elektrodenleitung (126, 218, 219), die eine Mehrzahl von Elektroden (132, 216) aufweist, Elektrostimulation an ein neurales Ziel des Patienten bereitzustellen; und
ein Programmiergerät (400), das kommunikativ mit dem implantierbaren Stimulator (127, 210) gekoppelt ist, wobei das Programmiergerät (402) einen Controller (402) aufweist, der dazu konfiguriert ist,
einen Suchraum von Elektrodenkonfigurationen und Parameterwerten für die Elektrodenleitung in Bezug auf das neurale Ziel zu identifizieren, wobei der Suchraum eine Teilmenge der Mehrzahl von Elektroden zur Abgabe von Stimulation sowie Stimulationsparameterwerte oder Wertebereiche die mit der Teilmenge der Elektroden in Verbindung sind, aufweist;
in einem ersten Patientenzustand einen klinischen Response-Indikator zu bewerten, der auf Elektrostimulationreagiert, die unter Verwendung von Elektroden und Stimulationsparameterwerten aus dem identifizierten Suchraum abgegeben wird, und zumindest eine Basis-Stimulationseinstellung zu bestimmen, die dem klinischen Response-Indikator entspricht, der eine spezifische Bedingung erfüllt, wobei die zumindest eine Basis-Stimulationseinstellung eine optimale Elektrodenkonfiguration und einen optimalen Stimulationsparameterwert aufweist; und
im Speicher (404) den identifizierten Suchraum und die zumindest eine Basis-Stimulationseinstellung zu speichern, die mit dem entsprechenden klinischen Response-Indikator verbunden ist; und
ein Therapietitrationsgerät (710), das dazu konfiguriert ist,
in einem zweiten Patientenzustand, der sich vom ersten Patientenzustand unterscheidet, den klinischen Response-Indikator zu bewerten, der auf Elektrostimulation gemäß der zumindest einen Basis-Stimulationseinstellung reagiert;
den klinischen Response-Indikator auf Grundlage eines Vergleichs zwischen dem klinischen Response-Indikator in dem zweiten Patientenzustand und dem klinischen Response-Indikator in dem ersten Patientenzustand zu modifizieren;
die zumindest eine Basis-Stimulationseinstellung zu modifizieren, indem die optimale Elektrodenkonfiguration oder der optimale Stimulationsparameterwert innerhalb des identifizierten Suchraums iterativ angepasst wird, bis der modifizierte klinische Response-Indikator eine spezifische Bedingung in dem zweiten Patientenzustand erfüllt;
im Speicher (404) die modifizierte zumindest eine Basis-Stimulationseinstellung zu speichern, die mit dem modifizierten klinischen Response-Indikator verbunden ist; und
ein Steuersignal an dem implantierbaren Stimulator zu erzeugen, um Elektrostimulation gemäß der modifizierten zumindest einen Basis-Stimulationseinstellung abzugeben.

2. System (100, 412) nach Anspruch 1, wobei das Therapietitrationsgerät (710) ein mobiles Gerät ist, das vom Patienten bedienbar ist und über eine drahtlose Verbindung mit dem implantierbaren Stimulator (127, 210) gekoppelt ist.

3. System (100, 412) nach einem der Ansprüche 1-2, wobei das Therapietitrationsgerät (710) eine Benutzeroberfläche aufweist, die dazu konfiguriert ist, den identifizierten Suchraum grafisch darzustellen und Benutzereingaben zum Modifizieren der zumindest einen Basis-Stimulationseinstellung zu empfangen.

4. System (100, 412) nach Anspruch 3, wobei die Benutzeroberfläche dazu konfiguriert ist, den identifizierten Suchraum grafisch als Heatmap darzustellen, die Intensitäten klinischer Reaktionen auf Elektrostimulation über einen Bereich von Elektrodenpositionen auf der Elektrodenleitung und einen Bereich von Stimulationsparameterwerten abbildet.

5. System (100, 412) nach einem der Ansprüche 1-4, wobei der Controller (402) dazu konfiguriert ist, den klinischen Response-Indikator unter Verwendung eines oder mehrerer klinischer Effekte zu bewerten, die auf Elektrostimulationreagieren, die unter Verwendung von Elektroden und Stimulationsparameterwerten aus dem identifizierten Suchraum abgegeben wird.

6. System (100, 412) nach Anspruch 5, wobei:
der Controller (402) dazu konfiguriert ist, den klinischen Response-Indikator unter Verwendung einer Kombination der einen oder mehreren klinischen Effekte , die jeweils mit entsprechenden Gewichtungsfaktoren gewichtet sind, zu bewerten; und
das Therapietitrationsgerät (710) dazu konfiguriert ist, auf Grundlage des Vergleichs zwischen dem klinischen Response-Indikator in dem zweiten Patientenzustand und dem klinischen Response-Indikator in dem ersten Patientenzustand einen oder mehrere der klinischen Effekte zu identifizieren, die sich vom ersten Patientenzustand zum zweiten Patientenzustand verschlechtert haben, und den klinischen Response-Indikator zu modifizieren, indem die entsprechenden Gewichtungsfaktoren für die identifizierten einen oder mehreren klinischen Effekte erhöht werden.

7. System (100, 412) nach einem der Ansprüche 5-6, wobei die einen oder mehreren klinischen Effekte Indikatoren einer therapeutischen Wirksamkeit oder einer Nebenwirkung der Elektrostimulation aufweisen.

8. System (100, 412) nach einem der Ansprüche 5-7, wobei die einen oder mehreren klinischen Effekte motorische Symptome aufweisen, einschließlich eines oder mehrerer von Bradykinese, Tremor oder Rigor.

9. System (100, 412) nach einem der Ansprüche 5-8, wobei das Therapietitrationsgerät (710) dazu konfiguriert ist, eine Benutzereingabe der einen oder mehreren klinischen Effekte zu empfangen.

10. System nach einem der Ansprüche 5-9, wobei das Therapietitrationsgerät (710) einen oder mehrere Sensoren aufweist oder kommunikativ mit diesem gekoppelt ist, um die einen oder mehreren klinischen Effekte zu erfassen.

11. System nach einem der Ansprüche 1-10, wobei der Controller (402) dazu konfiguriert ist, eine Mehrzahl von Basis-Stimulationseinstellungen festzustellen und im Speicher (404) zu speichern, die jeweils mit entsprechenden klinischen Response-Indikatoren verbunden sind, wobei die jeweiligen klinischen Response-Indikatoren jeweils in dem ersten Patientenzustand unter Verwendung eines oder mehrerer klinischer Effekte ausgewertet werden, die jeweils mit entsprechenden Gewichtungsfaktoren gewichtet sind, und wobei die Mehrzahl von Basis-Stimulationseinstellungen jeweils entsprechende optimale Elektrodenkonfigurationen und entsprechende optimale Stimulationsparameterwerte aufweist.

12. System nach Anspruch 11, wobei der Controller dazu konfiguriert ist, einen oder mehrere der Mehrzahl von klinischen Response-Indikatoren unter Verwendung gewichteter Kombinationen eines oder mehrerer klinischer Effekte , die jeweils mit entsprechenden Gewichtungsfaktoren gewichtet sind, zu bewerten, wobei die Gewichtungsfaktoren, die mit einer der Mehrzahl von Basis-Stimulationseinstellungen verbunden sind, sich von den Gewichtungsfaktoren unterscheiden, die mit einer anderen der Mehrzahl von Basis-Stimulationseinstellungen verbunden sind.

13. System nach einem der Ansprüche 11-12, wobei der Controller dazu konfiguriert ist,
in dem ersten Patientenzustand unter Verwendung eines trainierten Machine-Learning-Modells und ohne Abgabe von Elektrostimulation einen oder mehrere klinische Effekte für ungetestete Stimulationsparameterwerte oder ungetestete Elektrodenkonfigurationen in dem identifizierten Suchraum vorherzusagen;
einen klinischen Response-Indikator unter Verwendung der vorhergesagten einen oder mehreren klinischen Effekte zu bewerten; und
eine geschätzte Basis-Stimulationseinstellung zu bestimmen, die mit dem klinischen Response-Indikator verbunden ist, der unter Verwendung der vorhergesagten einen oder mehreren klinischen Effekte ausgewertet wird.

14. System nach einem der Ansprüche 11-13, wobei das Programmiergerät ein Therapietitrationsgerät (710) aufweist, das dazu konfiguriert ist,
in einem zweiten Patientenzustand, der sich vom ersten Patientenzustand unterscheidet, jeweilige klinische Response-Indikatoren zu bewerten, die auf Elektrostimulation gemäß der Mehrzahl von Basis-Stimulationseinstellungen reagieren;
auf Grundlage eines Vergleichs zwischen den klinischen Response-Indikatoren in dem zweiten Patientenzustand und den klinischen Response-Indikatoren in dem ersten Patientenzustand eine Basis-Stimulationseinstellung aus der Mehrzahl von Basis-Stimulationseinstellungen auszuwählen;
die ausgewählte Basis-Stimulationseinstellung zu modifizieren, indem eine optimale Elektrodenkonfiguration oder ein optimaler Stimulationsparameterwert der ausgewählten Basis-Stimulationseinstellung innerhalb des identifizierten Suchraums iterativ angepasst wird, bis der klinische Response-Indikator, der mit ausgewählten Basis-Stimulationseinstellung verbunden ist, eine spezifische Bedingung unter dem zweiten Patientenzustand erfüllt;
im Speicher (404) die modifizierte ausgewählte Basis-Stimulationseinstellung zu speichern; und
ein Steuersignal an den implantierbaren Stimulator (127, 210) zu erzeugen, um Elektrostimulation gemäß der modifizierten ausgewählten Basis-Stimulationseinstellung abzugeben.

## Revendications

1. Système (100, 412) destiné à fournir une électrostimulation à un patient, comprenant :
un stimulateur implantable (127, 210) configuré pour fournir une électrostimulation à une cible neuronale du patient via un fil (126, 218, 219) comprenant une pluralité d'électrodes (132, 216) ; et
un dispositif de programmation (400) couplé en communication au stimulateur implantable (127, 210), le dispositif de programmation (402) comportant un dispositif de commande (402) configuré pour :
identifier un espace de recherche de configurations d'électrodes et de valeurs de paramètres pour le fil par rapport à la cible neuronale, l'espace de recherche comportant un sous-ensemble de la pluralité d'électrodes destinées à administrer une stimulation et de valeurs ou de plages de valeurs de paramètres de stimulation associées au sous-ensemble d'électrodes ;
dans un premier état du patient, évaluer un indicateur de réponse clinique en réponse à une électrostimulation administrée à l'aide des électrodes et des valeurs de paramètres de stimulation à partir de l'espace de recherche identifié, et déterminer au moins un paramètre de stimulation de base qui correspond à l'indicateur de réponse clinique qui satisfait une condition spécifique, l'au moins un paramètre de stimulation de base comportant une configuration d'électrodes optimale et une valeur de paramètre de stimulation optimale ; et
stocker dans une mémoire (404) l'espace de recherche identifié et l'au moins un paramètre de stimulation de base associé à l'indicateur de réponse clinique correspondant ; et
un dispositif de titrage de thérapie (710) configuré pour :
dans un deuxième état du patient différent du premier état du patient, évaluer l'indicateur de réponse clinique en réponse à l'électrostimulation selon l'au moins un paramètre de stimulation de base ;
modifier l'indicateur de réponse clinique sur la base d'une comparaison entre l'indicateur de réponse clinique dans le deuxième état du patient et l'indicateur de réponse clinique dans le premier état du patient ;
modifier l'au moins un paramètre de stimulation de base en ajustant de manière répétée la configuration d'électrodes optimale ou la valeur de paramètre de stimulation optimale dans l'espace de recherche identifié jusqu'à ce que l'indicateur de réponse clinique modifié satisfasse une condition spécifique dans le deuxième état du patient ;
stocker dans la mémoire (404) l'au moins un paramètre de stimulation de base modifié associé à l'indicateur de réponse clinique modifié ; et
générer un signal de commande vers le stimulateur implantable afin d'administrer une électrostimulation selon l'au moins un paramètre de stimulation de base modifié.

2. Système (100, 412) selon la revendication 1, dans lequel le dispositif de titrage de thérapie (710) est un dispositif mobile qui peut être actionné par le patient et couplé au stimulateur implantable (127, 210) via une liaison sans fil.

3. Système (100, 412) selon l'une quelconque des revendications 1 à 2, dans lequel le dispositif de titrage de thérapie (710) comporte une interface utilisateur configurée pour afficher graphiquement l'espace de recherche identifié et pour recevoir une entrée utilisateur afin de modifier 'au moins un paramètre de stimulation de base.

4. Système (100, 412) selon la revendication 3, dans lequel l'interface utilisateur est configurée pour afficher graphiquement l'espace de recherche identifié sous la forme d'une carte de chaleur qui indique les intensités des réponses cliniques à l'électrostimulation sur une plage de positions d'électrodes sur le fil et une plage de valeurs de paramètres de stimulation.

5. Système (100, 412) selon l'une quelconque des revendications 1 à 4, dans lequel le dispositif de commande (402) est configuré pour évaluer l'indicateur de réponse clinique en utilisant un ou plusieurs effets cliniques en réponse à l'électrostimulation administrée à l'aide d'électrodes et les valeurs de paramètres de stimulation issues de l'espace de recherche identifié.

6. Système (100, 412) selon la revendication 5, dans lequel :
le dispositif de commande (402) est configuré pour évaluer l'indicateur de réponse clinique à l'aide d'une combinaison du ou des effets cliniques chacun pondérés par des facteurs de pondération respectifs ; et
le dispositif de titrage de thérapie (710) est configuré pour, sur la base de la comparaison entre l'indicateur de réponse clinique dans le deuxième état du patient et l'indicateur de réponse clinique dans le premier état du patient, identifier un ou plusieurs des effets cliniques qui se sont aggravés entre le premier état du patient et le deuxième état du patient, et pour modifier l'indicateur de réponse clinique en augmentant les facteurs de pondération respectifs pour le ou les effets cliniques identifiés.

7. Système (100, 412) selon l'une quelconque des revendications 5 à 6, dans lequel le ou les effets cliniques comprennent des indicateurs d'efficacité thérapeutique ou un effet secondaire de l'électrostimulation.

8. Système (100, 412) selon l'une quelconque des revendications 5 à 7, dans lequel le ou les effets cliniques comportent des symptômes moteurs comprenant un ou plusieurs d'une bradykinésie, de tremblements ou d'une rigidité.

9. Système (100, 412) selon l'une quelconque des revendications 5 à 8, dans lequel le dispositif de titrage de thérapie (710) est configuré pour recevoir une entrée utilisateur du ou des effets cliniques.

10. Système selon l'une quelconque des revendications 5 à 9, dans lequel le dispositif de titrage de thérapie (710) comporte, ou est couplé en communication à, un ou plusieurs capteurs destinés à détecter le ou les effets cliniques.

11. Système selon l'une quelconque des revendications 1 à 10, dans lequel le dispositif de commande (402) est configuré pour déterminer, et stocker dans la mémoire (404), une pluralité de paramètres de stimulation de base chacun associés à des indicateurs de réponse clinique respectifs, les indicateurs de réponse cliniques respectifs étant chacun évalués dans le premier état du patient à l'aide d'un ou plusieurs effets cliniques pondérés par les facteurs de pondération respectifs, les paramètres de stimulation de base comportant chacun des configurations d'électrodes optimales respectives et des valeurs de paramètres de stimulation optimales respectives.

12. Système selon la revendication 11, dans lequel le dispositif de commande est configuré pour évaluer un ou plusieurs de la pluralité d'indicateurs de réponse clinique à l'aide de combinaisons pondérées d'un ou plusieurs effets cliniques chacun pondérés par des facteurs de pondération respectifs, les facteurs de pondération étant associés à l'un de la pluralité de paramètres de stimulation de base différents des facteurs de pondération associés à un autre de la pluralité de paramètres de stimulation de base.

13. Système selon l'une quelconque des revendications 11 à 12, dans lequel le dispositif de commande est configuré pour :
dans le premier état du patient, prédire, à l'aide d'un modèle d'apprentissage machine formé et sans administrer d'électrostimulation, un ou plusieurs effets cliniques pour des valeurs de paramètres de stimulation non testées ou des configurations d'électrodes non testées dans l'espace de recherche identifié ;
évaluer un indicateur de réponse clinique à l'aide du ou des effets cliniques prédits ; et
déterminer un paramètre de stimulation de base estimé associé à l'indicateur de réponse clinique évalué à l'aide du ou des effets cliniques prédits.

14. Système selon l'une quelconque des revendications 11 à 13, dans lequel le dispositif de programmation comporte un dispositif de titrage de thérapie (710) configuré pour :
dans un deuxième état du patient différent du premier état du patient, évaluer des indicateurs de réponse clinique respectifs en réponse à une électrostimulation selon la pluralité de paramètres de stimulation de base ;
sur la base d'une comparaison entre les indicateurs de réponse clinique dans le deuxième état du patient et les indicateurs de réponse clinique dans le premier état du patient, sélectionner un paramètre de stimulation de base parmi la pluralité de paramètres de stimulation de base ;
modifier le paramètre de stimulation de base sélectionné en ajustant de manière répétée une configuration d'électrodes optimale ou une valeur de paramètre de stimulation optimale du paramètre de stimulation de base sélectionné dans l'espace de recherche identifié jusqu'à ce que l'indicateur de réponse clinique associé au paramètre de stimulation de base sélectionné satisfasse une condition spécifique dans le deuxième état du patient ;
stocker dans la mémoire (404) le paramètre de stimulation de base sélectionné ;
et
générer un signal de commande vers le stimulateur implantable (127, 210) afin d'administrer une électrostimulation selon le paramètre de stimulation de base sélectionné modifié.
